# EUROPEAN PATENT APPLICATION

(11) **EP 4 818 221 A1**
(43) Date of publication of application: **07.10.2026**
(21) Application number: 24907380.0
(22) Date of filing: 16.12.2024
(51) Int. Cl.: C08F 297/00, C12M 3/00

(54) **TEMPERATURE-RESPONSIVE BLOCK COPOLYMER, METHOD FOR PRODUCING SAME, AND CELL CULTURE SUBSTRATE**

(30) Priority: 22.12.2023 JP 2023216936
(71) Applicant: Tosoh Corporation, Shunan-shi, Yamaguchi 746-8501 (JP)
(72) Inventor: KINOSHITA, Yuya, Yokkaichi-shi, Mie 510-8540 (JP)
(74) Representative: Novagraaf International SA
(86) International application number: PCT/JP2024/044473
(87) International publication number: WO 2025/134995

(57) **Abstract**

The present disclosure relates to a temperature-responsive block copolymer containing a block segment (A) having a lower critical solution temperature (LCST) in water and a water-insoluble block segment (B), wherein, when a film containing the temperature-responsive block copolymer is formed, an amount of change in a bubble contact angle on a surface of the film, as measured under respective conditions in water at 21°C and in water at 37°C, is 5.0° or more.

## Description

### Technical Field

The present invention relates to a temperature-responsive block copolymer, a method for producing the same, and a cell culture substrate.

### Background Art

Temperature-responsive polymers have been studied for use in various applications such as cell culture substrates (see, for example, Patent Literatures 1 to 3). According to a cell culture substrate in which a surface of a substrate is coated with a temperature-responsive polymer, the adhesive force of the substrate surface can be weakened by a sol transition of the temperature-responsive polymer caused by a temperature drop in the surrounding environment, thereby allowing cells to be detached. This makes it possible to collect cells while suppressing damage to the cells.

### Citation List

### Patent Literature

[Patent Literature 1] Japanese Unexamined Patent Publication No. 2020144453
[Patent Literature 2] Japanese Patent No. 6447787
[Patent Literature 3] Japanese Patent No. 7293683

### Summary of Invention

### Technical Problem

An object of the present invention is to provide a temperature-responsive block copolymer capable of forming a cell culture substrate with improved cell detachability, and a method for producing the same. An object of the present invention is to provide a cell culture substrate with improved cell detachability.

### Solution to Problem

The present invention relates to the following inventions.
[1] A temperature-responsive block copolymer comprising a block segment (A) having a lower critical solution temperature (LCST) in water and a water-insoluble block segment (B), wherein, when a film comprising the temperature-responsive block copolymer is formed, an amount of change in a bubble contact angle on a surface of the film, as measured under respective conditions in water at 21°C and in water at 37°C, is 5.0° or more.
[2] The temperature-responsive block copolymer according to [1], wherein the amount of change in the bubble contact angle is 10° or more and 40° or less.
[3] The temperature-responsive block copolymer according to [1] or [2], wherein at least one functional group selected from the group consisting of a hydroxyl group, a mercapto group, an amino group, a substituted amino group, a cyano group, an ester group, an amide group, a carbonyl group, and a carboxyl group is introduced into at least one terminal of the block segment (A) or the block segment (B).
[4] The temperature-responsive block copolymer according to any one of [1] to [3], wherein at least one functional group selected from the group consisting of a hydroxyl group, a mercapto group, an amino group, a substituted amino group, a cyano group, an ester group, an amide group, a carbonyl group, and a carboxyl group is introduced into a terminal on the block segment (A) side.
[5] The block copolymer according to any one of [1] to [4], wherein the block segment (B) comprises a monomer unit represented by the following General Formula (b1). 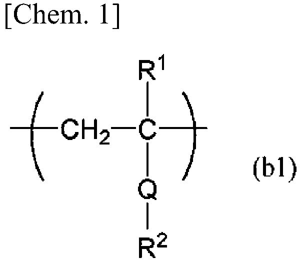 [In the formula, R¹ represents a hydrogen atom or a methyl group, Q represents an ester group, and R² represents a hydrocarbon group having 1 to 30 carbon atoms.]
[6] The temperature-responsive block copolymer according to any one of [1] to [5], wherein a degree of polymerization of the block segment (A) is 100 or more and less than 400.
[7] A cell culture substrate comprising a substrate and a coating layer covering at least a part of a surface of the substrate, wherein the coating layer comprises a temperature-responsive block copolymer, the temperature-responsive block copolymer comprises a block segment (A) having a lower critical solution temperature (LCST) in water and a water-insoluble block segment (B), and an amount of change in a bubble contact angle on a surface of the coating layer, as measured under respective conditions in water at 21°C and in water at 37°C, is 5.0° or more.
[8] The cell culture substrate according to [7], wherein a layer thickness of the coating layer is 5 to 1000 nm.
[9] The cell culture substrate according to [7] or [8], wherein the substrate is any one of a plate, a film, a flask, and a bag.
[10] The cell culture substrate according to any one of [7] to [9], comprising a region having cell adhesiveness and temperature-responsive detachability, and a region of non-cell adhesiveness.
[11] The cell culture substrate according to [10], wherein the region of non-cell adhesiveness comprises a hydrophilic polymer.
[12] The cell culture substrate according to any one of [7] to [11], wherein the coating layer has a phase-separated structure, or a surface structure derived from phase separation, at a culture temperature.
[13] The cell culture substrate according to any one of [7] to [12], wherein a thickness of the coating layer is 10 to 50 nm.
[14] The cell culture substrate according to any one of [7] to [13], wherein the substrate is polycarbonate.
[15] A method for producing a temperature-responsive block copolymer comprising a block segment (A) having a lower critical solution temperature (LCST) in water and a water-insoluble block segment (B), wherein a polar group is introduced into a terminal of the block segment (A) or (B), the method comprising: a step of reacting a thiocarbonylthio group-containing block copolymer comprising the block segments (A) and (B), and a terminal bonded to the block segment (A) or (B) comprising a thiocarbonylthio group represented by the following Formula (I): 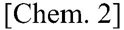 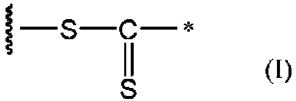
   [In Formula (I), a wavy line indicates a bond on the block segment (A) or (B) side, and * indicates a bond on a terminal side.]
   with a radical generator having a polar group to convert the terminal comprising the thiocarbonylthio group into a terminal comprising the polar group, thereby obtaining the temperature-responsive block copolymer.
[16] The method according to [15], wherein the radical generator having a polar group comprises at least one selected from the group consisting of azobisisobutyronitrile, 2,2'-azobis[2-methyl-N-(2-hydroxyethyl)propionamide], 4,4'-azobis-(4-cyanovaleric acid), and 2,2'-azobis-(2-methylpropionic acid).
[17] A method for producing a temperature-responsive block copolymer comprising a block segment (A) having a lower critical solution temperature (LCST) in water and a water-insoluble block segment (B), wherein a mercapto group is introduced into a terminal of the block segment (A) or (B), the method comprising: a step of hydrolyzing a thiocarbonylthio group-containing block copolymer comprising the block segments (A) and (B), and a terminal bonded to the block segment (A) or (B) comprising a thiocarbonylthio group represented by the following Formula (I):

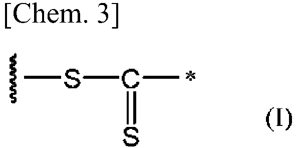

[In Formula (I), a wavy line indicates a bond on the block segment (A) or (B) side, and * indicates a bond on a terminal side.]
to convert the terminal comprising the thiocarbonylthio group into a mercapto group, thereby obtaining the temperature-responsive block copolymer.

### Advantageous Effects of Invention

According to the present invention, it is possible to provide a temperature-responsive block copolymer capable of forming a cell culture substrate with improved cell detachability, and a method for producing the same. According to the present invention, it is possible to provide a cell culture substrate with improved cell detachability.

### Description of Embodiments

In the present specification, a numerical range indicated using "to" represents a range that includes the numerical values described before and after "to" as the minimum value and the maximum value, respectively. Unless otherwise specified, the units of the numerical values described before and after "to" are the same. In numerical ranges described in a stepwise manner in the present specification, the upper limit value or the lower limit value of a numerical range at one step may be replaced with the upper limit value or the lower limit value of a numerical range at another step. In addition, in the numerical ranges described in the present specification, the upper limit value or the lower limit value of the numerical range may be replaced with a value shown in the Examples. Furthermore, the upper limit values and the lower limit values described individually may be combined in any manner. The materials exemplified below may be used alone or in combination of two or more types, unless otherwise specified. The content of each component in a composition means the total amount of the plurality of substances corresponding to each component present in the composition, unless otherwise specified, when a plurality of substances corresponding to each component are present in the composition. In the present specification, "and/or" means either or both. For example, "A and/or B" means any one of A, B, and a combination of A and B.

Hereinafter, embodiments for carrying out the present invention will be described in detail. The present invention is not limited to the following embodiments, and the following embodiments may be modified and carried out.

### [Block Copolymer]

The temperature-responsive block copolymer according to the present embodiment includes a temperature-responsive block segment (A) having a lower critical solution temperature (LCST) in water and a water-insoluble block segment (B). In the present specification, the "temperature-responsive block copolymer" refers to a polymer in which the degree of hydrophilicity and hydrophobicity changes with a change in temperature. The temperature-responsive block copolymer is a polymer that changes to be more hydrophobic beyond a specific temperature when the temperature of the polymer is raised from a low temperature to a high temperature in the presence of water molecules.

When a film comprising the temperature-responsive block copolymer is formed, an amount of change in a bubble contact angle on a surface of the film, as measured under respective conditions in water at 21°C and in water at 37°C, is 5.0° or more. By forming a coating layer containing the temperature-responsive block copolymer on a cell culture substrate, a cell culture substrate with improved cell detachability can be obtained. In the present specification, "cell detachability" refers to the ease of detachment of cells that have proliferated on a cell culture substrate from the cell culture substrate. The film may be any film containing the temperature-responsive block copolymer. The film is preferably a film containing 80% by mass or more of the temperature-responsive block copolymer, and more preferably a film consisting solely of the temperature-responsive block copolymer.

The amount of change in the bubble contact angle is determined from the bubble contact angle measured by the following method. The temperature-responsive block copolymer is diluted with ethanol to prepare a polymer solution. The content of the temperature-responsive block copolymer in the polymer solution is 0.8% by mass based on the total amount of the polymer solution. 100 µL of the polymer solution is dropped onto a polycarbonate film, and a film comprising the temperature-responsive block copolymer is formed on the polycarbonate film by spin coating at a rotation speed of 2000 rpm for 60 seconds. The polycarbonate film provided with the formed film is used as a film for bubble contact angle measurement. The film for bubble contact angle measurement is immersed in water at 21°C or 37°C for 10 minutes, and the bubble contact angle (°) is measured using a contact angle meter under conditions of in water and a bubble of 1 µL. The amount of change in the bubble contact angle is determined by calculating the difference (θ₁ - θ₂) between the bubble contact angle (θ₁) measured in water at 21°C and the bubble contact angle (θ₂) measured in water at 37°C. The detailed measurement conditions are as described in the Examples described later.

The amount of change in the bubble contact angle may be 8.0° or more, and may be 10.0° or more, 12.0° or more, 14.0° or more, 16.0° or more, 18.0° or more, 20.0° or more, 22.0° or more, 24.0° or more, 26.0° or more, 28.0° or more, or 30.0° or more, since cell detachability is further improved. The amount of change in the bubble contact angle may be 40.0° or less, 38.0° or less, 36.0° or less, 34.0° or less, 32.0° or less, 30.0° or less, 28.0° or less, 26.0° or less, 24.0° or less, 22.0° or less, 20.0° or less, 18.0° or less, 16.0° or less, 14.0° or less, or 12.0° or less. The amount of change in the bubble contact angle may be 10.0° or more and 40.0° or less, 14.0° or more and 36.0° or less, 18.0° or more and 32.0° or less, or 20.0° or more and 30.0° or less, since cell detachability is further improved.

The bubble contact angle (θ₁) measured in water at 21°C may be, for example, 140° or more, 145° or more, 150° or more, 152° or more, or 154° or more, and may be 170° or less, 165° or less, or 161° or less.

The bubble contact angle (θ₂) measured in water at 37°C may be, for example, 120° or more, 125° or more, or 130° or more, and may be 160° or less, 155° or less, or 150° or less.

The amount of change in the bubble contact angle can be adjusted to satisfy the numerical ranges described above by, for example, adjusting the type and content of the block segment (A) in the block copolymer, adjusting the degree of hydrophilicity or hydrophobicity of the functional group present in the block segment (A) (for example, introducing a polar group into the terminal of the block segment (A)), adjusting the ratio of the block segment (A) to the block segment (B), adjusting the layer thickness of the coating layer of the block copolymer, and a combination thereof.

The temperature-responsive block copolymer may be a diblock copolymer containing at least the block segment (A) and the block segment (B). The block segment (A) and the block segment (B) may be directly bonded to each other or may be bonded via a spacer.

In the temperature-responsive block copolymer, a polar group may be introduced into either terminal of the block segment (A) or the block segment (B). Examples of the polar group include a hydroxyl group (-OH), a mercapto group (-SH), an amino group (-NH₂), a substituted amino group (a monosubstituted amino group (-NHR) or a disubstituted amino group (-NR₂)), a cyano group (-CN), an ester group (-C(=O)-O-R, or -O-C(=O)-R), an amide group (-NH-C(=O)-R, or-C(=O)-NH-R), a carbonyl group (-C(=O)-R), a carboxyl group (-COOH), a nitro group (-NO₂), and a sulfo group (-SO₃H). R represents a substituent in the polar group. R may be, for example, an alkyl group, and may be an alkyl group having 1 to 6 or 1 to 3 carbon atoms.

The polar group is preferably a nitrile group, a hydroxyl group, a carboxyl group, an ester group (for example, -C(=O)-O-CH₃), or a mercapto group, and is more preferably a nitrile group, a hydroxyl group, a carboxyl group, or an ester group, since reactions between terminals or reactions with other substrates are less likely to occur (excellent terminal stability).

In the temperature-responsive block copolymer, the introduction of a polar group at the terminal can be confirmed by nuclear magnetic resonance spectroscopy (¹H-NMR spectral analysis and ¹³C-NMR spectral analysis) and by Fourier transform infrared spectroscopy (FT-IR).

The number-average molecular weight (Mn) of the temperature-responsive block copolymer may be 8,000 or more, 10,000 or more, 15,000 or more, 20,000 or more, 25,000 or more, 30,000 or more, or 35,000 or more, and may be 200,000 or less, 150,000 or less, 100,000 or less, 80,000 or less, 60,000 or less, or 45,000 or less.

The dispersity (Mw/Mn) of the temperature-responsive block copolymer may be 1.30 or less, 1.25 or less, 1.20 or less, or 1.15 or less, since cell detachability is further improved. The dispersity (Mw/Mn) of the temperature-responsive block copolymer may be, for example, 1.02 or more, 1.06 or more, 1.08 or more, 1.10 or more, 1.12 or more, or 1.14 or more.

The weight-average molecular weight (Mw), number-average molecular weight (Mn), and molecular weight distribution (Mw/Mn) are measured by gel permeation chromatography (GPC). The GPC apparatus used is HLC-8320GPC manufactured by Tosoh Corporation, and two columns of TSKgel Super AWM-H manufactured by Tosoh Corporation are used. The column temperature is set to 40°C. N,N-dimethylformamide containing 10 mM lithium bromide is used as the eluent. The measurement sample is prepared at 1.0 mg/mL. A calibration curve for molecular weight is prepared using polymethyl methacrylate of known molecular weight (manufactured by Polymer Laboratories Ltd.).

The block segment (A) is a temperature-responsive block segment having a lower critical solution temperature (LCST) in water.

The LCST of the block segment (A) refers to the LCST of a polymer containing only the monomers constituting the monomer units of the block segment (A) as monomer units (hereinafter also referred to as "polymer (A)"). The LCST of the block segment (A) is the temperature at which the polymer (A) becomes insoluble in water. The LCST is a value determined by measuring the transmittance of light at a wavelength of 500 nm while raising the temperature of an aqueous solution in which the polymer (A) is dissolved at 0.6% by mass at a rate of 1°C/min. The transmittance is approximately constant from a low temperature until the LCST is reached. Since the aqueous solution becomes turbid near the LCST, the transmittance drops sharply, and thereafter the transmittance becomes approximately constant again. The LCST can be determined by finding the temperature at which the transmittance is the average of the transmittance at temperatures below the LCST and the transmittance at temperatures above the LCST from the curve obtained from a graph plotting the change in transmittance with temperature change (midpoint method). The temperature range to be measured includes a temperature range of 5°C or more in which the transmittance is approximately constant at temperatures below the LCST, and further includes a temperature range of 5°C or more in which the transmittance is constant at temperatures above the LCST. The LCST can be specifically determined by the method described in Patent Literature 3.

Since cells can be cultured at a temperature close to body temperature when a substrate coated with the block copolymer is used for cell culture, the LCST of the block segment (A) is preferably 50°C or less, more preferably 35°C or less, and preferably 25°C or less from the viewpoint of further suppressing detachment of cells accompanying operations such as medium exchange during culture. The LCST of the block segment (A) is preferably 0°C or more, 5°C or more, 10°C or more, or 15°C or more, since cells can be detached under conditions that further suppress damage to cells.

The monomer constituting the monomer units of the block segment (A) is not particularly limited, and examples thereof include (meth)acrylamide compounds such as acrylamide and methacrylamide; N-alkyl-substituted (meth)acrylamide derivatives such as N,N-diethylacrylamide, N-ethylacrylamide, N-n-propylacrylamide, N-n-propylmethacrylamide, N-isopropylacrylamide, N-isopropylmethacrylamide, N-cyclopropylacrylamide, N-cyclopropylmethacrylamide, N-t-butylacrylamide, N-ethoxyethylacrylamide, N-ethoxyethylmethacrylamide, N-tetrahydrofurfurylacrylamide, and N-tetrahydrofurfurylmethacrylamide; N,N-dialkyl-substituted (meth)acrylamide derivatives such as N,N-dimethyl(meth)acrylamide, N,N-ethylmethylacrylamide, and N,N-diethylacrylamide; (meth)acrylamide derivatives having a cyclic group such as 1-(1-oxo-2-propenyl)-pyrrolidine, 1-(1-oxo-2-propenyl)-piperidine, 4-(1-oxo-2-propenyl)-morpholine, 1-(1-oxo-2-methyl-2-propenyl)-pyrrolidine, 1-(1-oxo-2-methyl-2-propenyl)-piperidine, and 4-(1-oxo-2-methyl-2-propenyl)-morpholine; vinyl ethers such as methyl vinyl ether; and proline derivatives such as N-prolinemethyl ester acrylamide.

The monomer constituting the monomer units of the block segment (A) is preferably, for example, at least one selected from the group consisting of N,N-diethylacrylamide, N-n-propylacrylamide, N-isopropylacrylamide, N-n-propylmethacrylamide, N-ethoxyethylacrylamide, N-tetrahydrofurfurylacrylamide, and N-tetrahydrofurfurylmethacrylamide, more preferably at least one selected from the group consisting of N-n-propylacrylamide and N-isopropylacrylamide, and still more preferably N-isopropylacrylamide.

The block segment (A) may contain one type or two or more types of monomers as monomer units. The arrangement of the block segment (A) containing two or more types of monomers as monomer units may be any of a random arrangement, an alternating arrangement, and a block arrangement.

The degree of polymerization of the block segment (A) in the temperature-responsive block copolymer may be 100 or more, 120 or more, 140 or more, 160 or more, 180 or more, 200 or more, 210 or more, 220 or more, or 230 or more, since cell detachability is improved. The degree of polymerization of the block segment (A) in the block copolymer may be less than 400, 350 or less, 300 or less, 280 or less, 260 or less, 250 or less, or 240 or less, since elution into water is less likely to occur when used as a coating material for a cell culture substrate. The degree of polymerization of the block segment (A) in the temperature-responsive block copolymer is preferably 200 to 350, since cell detachability is improved. The degree of polymerization of the block segment (A) can be calculated by, for example, the following formula. Formula: 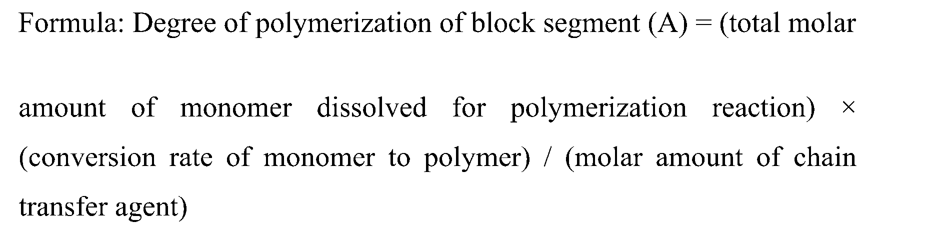 Formula: Degree of polymerization of block segment (A) = (total molaramount of monomer dissolved for polymerization reaction) × amount of monomer dissolved for polymerization reaction) × (conversion rate of monomer to polymer) / (molar amount of chain transfer agent) Here, the conversion rate of the monomer to the polymer is calculated by ¹H-NMR measurement of the reaction solution immediately after the polymerization reaction is stopped. Specifically, it can be calculated by the following formula using the integral value of a proton at a specific position of the monomer or polymer. 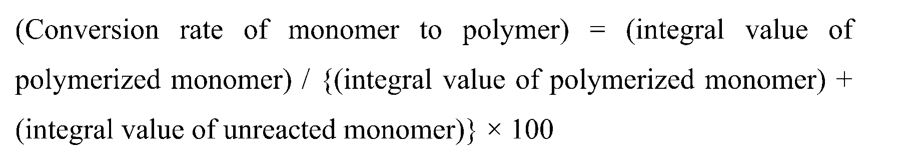 (Conversion rate of monomer to polymer) = (integral value of polymerized monomer) / {(integral value of polymerized monomer) + (integral value of unreacted monomer)} × 100

The degree of polymerization of the block segment (A) can also be calculated from the copolymer composition ratio determined by analyzing the block copolymer by pyrolysis gas chromatography and the absolute molecular weight of the block copolymer.

The proportion of the block segment (A) in the temperature-responsive block copolymer may be 50% by mass or more, 55% by mass or more, 60% by mass or more, or 65% by mass or more, based on the total amount of the temperature-responsive block copolymer. The proportion of the block segment (A) in the block copolymer may be 95% by mass or less, 90% by mass or less, 85% by mass or less, or 80% by mass or less. The proportion of the block segment (A) can be calculated by, for example, the following formula. Formula: 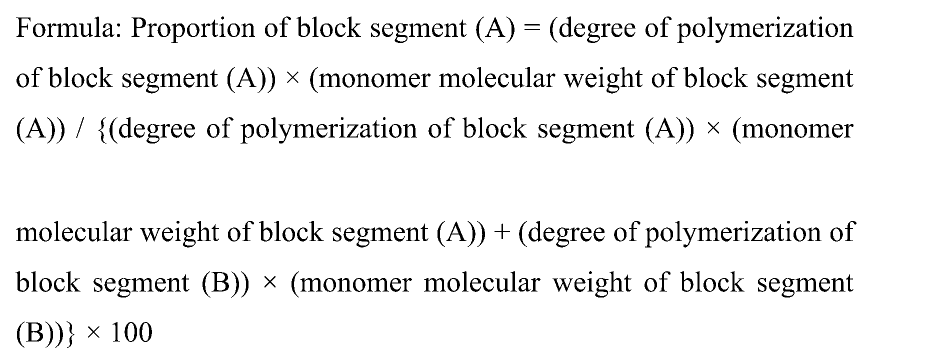 Proportion of block segment (A) = (degree of polymerization of block segment (A)) × (monomer molecular weight of block segment (A)) / {(degree of polymerization of block segment (A)) × (monomer molecular weight of block segment (A)) + (degree of polymerization of block segment (B)) × (monomer molecular weight of block segment (B))} × 100

It is preferable that a polar group is introduced into the terminal of the block segment (A). In this case, cell detachability is further improved. It is preferable that at least one functional group selected from the group consisting of a hydroxyl group, a mercapto group, an amino group, a substituted amino group, a cyano group, an ester group, an amide group, a carbonyl group, and a carboxyl group is introduced into the terminal of the block segment (A), since cell detachability is further improved. When the degree of polymerization of the block segment (A) is 100 or more and less than 400, the polar group at the terminal on the block segment (A) side is preferably at least one selected from the group consisting of a hydroxyl group, an amino group, a substituted amino group, a cyano group, an ester group, an amide group, a carbonyl group, and a carboxyl group, from the viewpoint of further improving cell detachability. In this case, it is considered that the block segment (A) is more likely to be arranged on the side opposite to the substrate (the water side) in water.

The block copolymer may contain, as the block segment (A) and a terminal group bonded to the block segment (A), a structure represented by, for example, the following General Formula (a1).

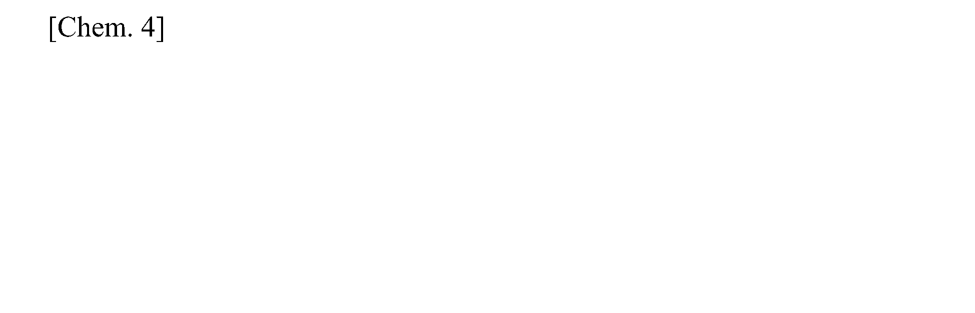

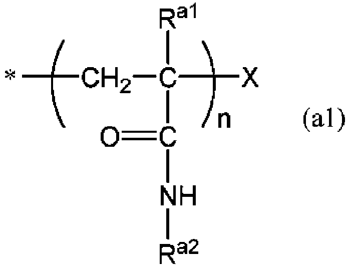

In Formula (a1), R^{a1} represents a hydrogen atom or a methyl group. R^{a2} represents an alkyl group. The alkyl group represented by R^{a2} may be branched or linear. The alkyl group represented by R^{a2} may be an alkyl group having 1 to 3 carbon atoms, or may be an isopropyl group. n represents an integer of 1 or more. * represents a bonding site with segment (B).

X represents a group comprising a polar group. X may comprise one or two or more polar groups. X may be a functional group consisting of a polar group and a linking group that links the block segment (A) and the polar group, or a functional group consisting only of a polar group. Examples of X include -C(CH₃)₂-CN, -C(CH₃)₂-C(=O)NHCH₂CH₂-OH, -C(CH₃)₂-C(=O)OCH₃, -C(CH₃)(CN)-CH₂CH₂-COOH, and -SH.

The block segment (B) is a water-insoluble block segment. The block segment (B) indicates that a polymer containing only the monomers constituting the monomer units of the block segment (B) as monomer units (hereinafter also referred to as "polymer (B)") is insoluble in water. The term "insoluble in water" means that the amount dissolved in 100 g of water is less than 0.01 g at 25°C. The amount of the polymer (B) dissolved in 100 g of water is preferably 10 mg or less.

The block segment (B) may contain one type or two or more types of monomers as monomer units. The arrangement of the block segment (B) containing two or more types of monomers as monomer units may be any of a random arrangement, an alternating arrangement, and a block arrangement.

The block segment (B) may contain, for example, a monomer unit represented by the following Formula (b1) (hereinafter also referred to as "monomer unit (b1)").

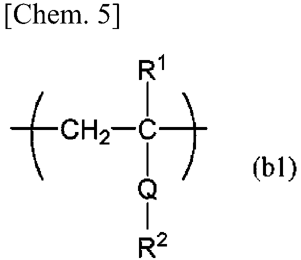

In Formula (b1), R¹ represents a hydrogen atom or a methyl group.

Q represents a divalent group selected from the group consisting of an ester group (*1-C(=O)-O-*2), an amide group (*1-C(=O)-NH-*2), a urethane group (*1-NH-C(=O)-O-*2 or *1-O-C(=O)-NH-*2), and an ether group (-O-). *2 represents a bonding site with R², and *1 represents a bonding site with a carbon atom. Q is preferably an ester group or an amide group, and more preferably an ester group.

R² represents a hydrocarbon group having 1 to 30 carbon atoms, a substituent represented by the following General Formula (b2) (hereinafter also referred to as "substituent (b2)"), a substituent represented by the following General Formula (b3) (hereinafter also referred to as "substituent (b3)"), or a hydrogen atom.

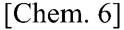

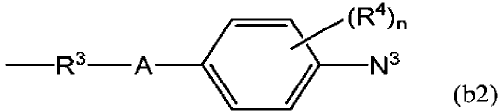

In Formula (b2), A represents an ether group or an ester group, R³ represents a divalent hydrocarbon group having 1 to 5 carbon atoms, R⁴ represents a fluorine atom, and n represents an integer of 0 to 4.]

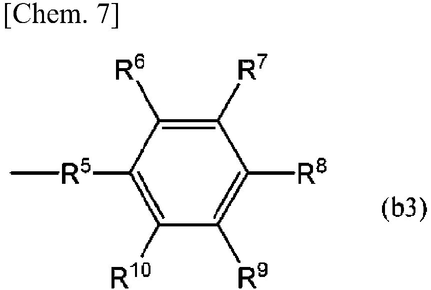

In Formula (b3), R⁵ represents a divalent hydrocarbon group having 1 to 5 carbon atoms or a single bond. R⁶, R⁷, R⁸, R⁹, and R¹⁰ each independently represent a hydrogen atom, a hydroxyl group, a carboxyl group, an amino group, or a hydrocarbon group having 1 to 4 carbon atoms.

The hydrocarbon group having 1 to 30 carbon atoms represented by R² may be linear or branched. The lower limit of the number of carbon atoms of the hydrocarbon group having 1 to 30 carbon atoms represented by R² may be 2 or more, 3 or more, or 4 or more. The upper limit of the number of carbon atoms of the hydrocarbon group may be 25 or less, 20 or less, 15 or less, 12 or less, 9 or less, 7 or less, or 5 or less. R² is preferably a hydrocarbon group having 4 to 15 carbon atoms, since water insolubility, adhesiveness to a substrate, and cell proliferativity are favorable.

The monomer constituting the monomer unit (b1) in which R² is a hydrocarbon group having 1 to 30 carbon atoms is not particularly limited, and examples thereof include n-butyl acrylate, n-butyl methacrylate, isobutyl acrylate, isobutyl methacrylate, t-butyl acrylate, t-butyl methacrylate, n-hexyl acrylate, n-hexyl methacrylate, n-octyl acrylate, n-octyl methacrylate, n-decyl acrylate, n-decyl methacrylate, n-dodecyl acrylate, n-dodecyl methacrylate, n-tetradecyl acrylate, and n-tetradecyl methacrylate. The monomer constituting the monomer unit (b1) preferably contains at least one monomer selected from the group consisting of n-butyl acrylate and n-butyl methacrylate, since water insolubility, adhesiveness to a substrate, and cell proliferativity are favorable, and the block copolymer can be more stably coated. When the monomer constituting the monomer unit (b1) is n-butyl methacrylate, a polymer with a smaller dispersity tends to be more easily obtained.

The monomer constituting the monomer unit (b1) in which R² is the substituent (b2) is not particularly limited, and examples thereof include 4-azidophenyl acrylate, 4-azidophenyl methacrylate, 2-((4-azidobenzoyl)oxy)ethyl acrylate, and 2-((4-azidobenzoyl)oxy)ethyl methacrylate.

The monomer constituting the monomer unit (b1) in which R² is the substituent (b3) is not particularly limited, and examples thereof include 2-hydroxyphenyl acrylate, 2-hydroxyphenyl methacrylate, 3-hydroxyphenyl acrylate, 3-hydroxyphenyl methacrylate, 4-hydroxyphenyl acrylate, 4-hydroxyphenyl methacrylate, N-(2-hydroxyphenyl)acrylamide, N-(2-hydroxyphenyl)methacrylamide, N-(3-hydroxyphenyl)acrylamide, N-(3-hydroxyphenyl)methacrylamide, N-(4-hydroxyphenyl)acrylamide, N-(4-hydroxyphenyl)methacrylamide, and styrene.

The block segment (B) may or may not contain monomer units other than the monomer unit (b1) (hereinafter also referred to as "other monomer units"). The monomer constituting the other monomer units is not particularly limited, and examples thereof include those having an amino group such as 2-dimethylaminoethyl acrylate, 2-dimethylaminoethyl methacrylate, 2-diethylaminoethyl acrylate, 2-diethylaminoethyl methacrylate, and N-[3-(dimethylamino)propyl]acrylamide; those having a betaine such as N-(3-sulfopropyl)-N-methacryloyloxyethyl-N,N-dimethylammonium betaine and N-methacryloyloxyethyl-N,N-dimethylammonium-α-N-methylcarboxybetaine; those having a polyethylene glycol group or a methoxyethyl group such as hydroxyethyl acrylate, hydroxyethyl methacrylate, N-(2-hydroxyethyl)acrylamide, polyethylene glycol monoacrylate, polyethylene glycol monomethacrylate, polypropylene glycol monoacrylate, polypropylene glycol monomethacrylate, methoxypolyethylene glycol monoacrylate, methoxypolyethylene glycol monomethacrylate, diethylene glycol monomethyl ether acrylate, diethylene glycol monomethyl ether methacrylate, diethylene glycol monoethyl ether acrylate, diethylene glycol monoethyl ether methacrylate, 2-methoxyethyl acrylate, 2-methoxyethyl methacrylate, 2-ethoxyethyl acrylate, 2-ethoxyethyl methacrylate, 3-butoxyethyl acrylate, 3-butoxyethyl methacrylate, 3-butoxyethyl acrylamide, furfuryl acrylate, furfuryl methacrylate, tetrahydrofurfuryl acrylate, and tetrahydrofurfuryl methacrylate; those having an acrylate group such as methoxymethyl acrylate, methoxymethyl methacrylate, 2-ethoxymethyl acrylate, 2-ethoxymethyl methacrylate, 3-butoxymethyl acrylate, 3-butoxymethyl methacrylate, and 3-butoxymethyl acrylamide; and those having a phosphorylcholine group such as 2-methacryloyloxyethyl phosphorylcholine, 2-acryloyloxyethyl phosphorylcholine, 3-(meth)acryloyloxypropyl phosphorylcholine, 4-(meth)acryloyloxybutyl phosphorylcholine, 6-(meth)acryloyloxyhexyl phosphorylcholine, 10-(meth)acryloyloxydecyl phosphorylcholine, ω-(meth)acryloyl(poly)oxyethylene phosphorylcholine, 2-acrylamidoethyl phosphorylcholine, 3-acrylamidopropyl phosphorylcholine, 4-acrylamidobutyl phosphorylcholine, 6-acrylamidohexyl phosphorylcholine, 10-acrylamidodecyl phosphorylcholine, and ω-(meth)acrylamido(poly)oxyethylene phosphorylcholine.

The proportion of the other monomer units may be 10% by mass or less, 5% by mass or less, 3% by mass or less, or 1% by mass or less, and may be 1% by mass or more, 3% by mass or more, or 5% by mass or more, based on the total amount of the block segment (B).

The degree of polymerization of the block segment (B) in the temperature-responsive block copolymer may be 20 or more, 40 or more, or 60 or more. The degree of polymerization of the block segment (B) in the block copolymer may be 160 or less, 140 or less, 120 or less, or 100 or less. The degree of polymerization of the block segment (B) in the temperature-responsive block copolymer is preferably 60 to 100. The degree of polymerization of the block segment (B) can be calculated by, for example, the following formula. Formula: 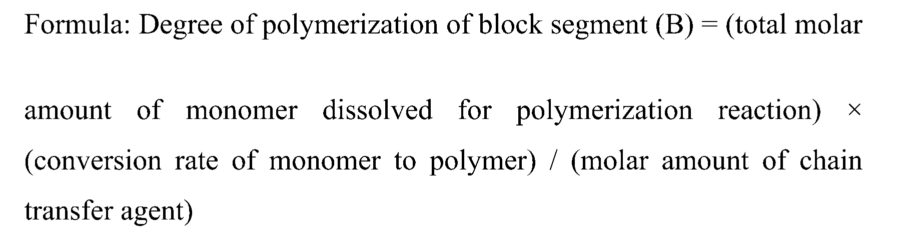 Degree of polymerization of block segment (B) = (total molar amount of monomer dissolved for polymerization reaction) × (conversion rate of monomer to polymer) / (molar amount of chain transfer agent)

The proportion of the block segment (B) in the temperature-responsive block copolymer may be 5% by mass or more, 10% by mass or more, 15% by mass or more, or 20% by mass or more, based on the total amount of the temperature-responsive block copolymer. The proportion of the block segment (B) in the block copolymer may be 50% by mass or less, 45% by mass or less, 40% by mass or less, or 35% by mass or less, based on the total amount of the temperature-responsive block copolymer. The proportion of the block segment (B) can be calculated by, for example, the following formula. Formula: 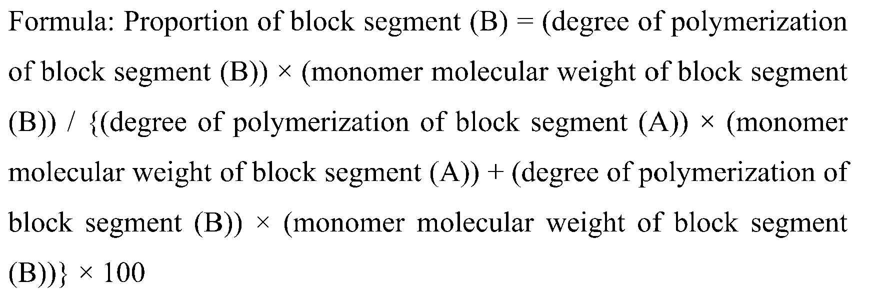 Proportion of block segment (B) = (degree of polymerization of block segment (B)) × (monomer molecular weight of block segment (B)) / {(degree of polymerization of block segment (A)) × (monomer molecular weight of block segment (A)) + (degree of polymerization of block segment (B)) × (monomer molecular weight of block segment (B))} × 100

The temperature-responsive block copolymer may contain a structure represented by the following Formula (C).

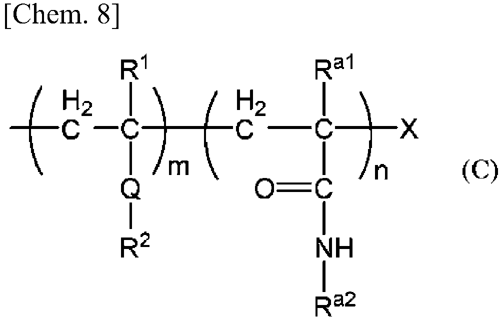

R^{a1}, R^{a2}, n, R¹, R², Q, and X may have the same definitions as described above. m represents an integer of 1 or more.

### [Method for Producing Temperature-Responsive Block Copolymer]

The temperature-responsive block copolymer can be obtained by, for example, a method including adjusting the amount of change in the bubble contact angle of a block copolymer containing the block segment (A) and the block segment (B) to 5.0° or more. The method for adjusting the amount of change in the bubble contact angle can be, for example, the method described above.

The polymerization method for the block copolymer is not particularly limited, and a common method for forming a copolymer can be employed. The polymerization method for the block copolymer may be radical polymerization, and may be, for example, reversible addition-fragmentation chain transfer (RAFT) polymerization, atom transfer radical polymerization (ATRP), nitroxide-mediated polymerization (NMP), or the like.

For the polymerization of the block copolymer, a chain transfer agent (RAFT agent), a polymerization initiator, a polymerization inhibitor, and the like may be used as necessary.

The RAFT agent is not particularly limited, and those commonly used can be suitably used; examples thereof include 4-cyano-4-[(dodecylsulfonylthiocarbonyl)sulfonyl]pentanoic acid, 4-[(2-carboxyethylsulfanylthiocarbonyl)sulfanyl]-4-cyanopentanoic acid, 2-{[(2-carboxyethyl)sulfanylthiocarbonyl]sulfanyl}propanoic acid, 2-cyano-2-[(dodecylsulfanylthiocarbonyl)sulfanyl]propane, 2-[(dodecylsulfanylthiocarbonyl)sulfanyl]propanoic acid, methyl 4-cyano-4-[(dodecylsulfanylthiocarbonyl)sulfanyl]pentanoate, 4-cyano-4-[(thiobenzoyl)sulfanyl]pentanoic acid, 2'-cyanobutane-2'-yl 4-chloro-3,5-dimethylpyrazole-1-carbodithioate, 2'-cyanobutane-2'-yl 3,5-dimethylpyrazole-1-carbodithioate, cyanomethyl 3,5-dimethylpyrazole-1-carbodithioate, S,S-dibenzyl trithiocarbonate, bis[4-(allyloxycarbonyl)benzyl] trithiocarbonate, bis[4-(2,3-dihydroxypropoxycarbonyl)benzyl] trithiocarbonate, bis{4-[ethyl-(2-acetyloxyethyl)carbamoyl]benzyl} trithiocarbonate, bis[4-(2-hydroxyethoxycarbonyl)benzyl] trithiocarbonate, dithiobenzoate, trithiocarbonate, 2-cyanopropan-2-yl N-methyl-N-(pyridin-4-yl)carbamodithioate, and methyl 2-(methyl(4-pyridinyl)carbamodithioate)propionate.

The polymerization initiator is not particularly limited, and those commonly used can be suitably used; examples thereof include azobisisobutyronitrile, 1,1'-azobis(cyclohexanecarbonitrile), 2,2'-azobis(4-methoxy-2,4-dimethylvaleronitrile), 2,2'-azobis(2,4-dimethylvaleronitrile), 2,2'-azobis(2-methylbutyronitrile), dimethyl 2,2'-azobis(2-methylpropionate), 2,2'-azobis(2-methylbutyronitrile), dimethyl 1,1'-azobis(1-cyclohexanecarboxylate), 2,2'-azobis[N-(2-carboxyethyl)-2-methylpropionamidine], 2,2'-azobis[2-methyl-N-(2-hydroxyethyl)propionamide], 4,4'-azobis-(4-cyanovaleric acid), di-tert-butyl peroxide, tert-butyl hydroperoxide, hydrogen peroxide, potassium peroxodisulfate, benzoyl peroxide, triethylborane, and diethylzinc.

The polymerization method for the block copolymer is preferably RAFT polymerization, since the desired temperature-responsive block copolymer is more easily obtained. RAFT polymerization is a radical-initiated polymerization reaction involving an exchange chain reaction utilizing a thiocarbonylthio group (-S-C(=S)-), and the product of the RAFT polymerization reaction usually has a thiocarbonylthio group.

The temperature-responsive block copolymer can be specifically produced by a method including a modification step of modifying a thiocarbonylthio group-containing block copolymer to obtain the temperature-responsive block copolymer. In the modification step, the amount of change in the bubble contact angle is adjusted to fall within the numerical ranges described above.

The thiocarbonylthio group-containing block copolymer comprises the block segments (A) and (B), and a terminal group bonded to the block segment (A) or (B) and containing a thiocarbonylthio group. The terminal group containing a thiocarbonylthio group is represented by the following Formula (I). 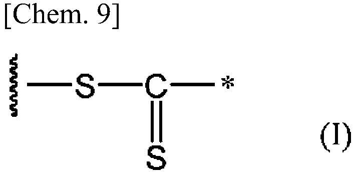 In Formula (I), a wavy line indicates a bond on the block segment (A) or (B) side, and * indicates a bond on a terminal side.

The thiocarbonylthio group-containing block copolymer can be obtained by, for example, a method comprising a polymerization step (1) of obtaining a thiocarbonylthio group-containing intermediate by a polymerization reaction of a first monomer in a mixture comprising the first monomer, a polymerization initiator, and a RAFT agent, and a polymerization step (2) of obtaining the thiocarbonylthio group-containing block copolymer by a reaction between the thiocarbonylthio group-containing intermediate and a second monomer in a mixture comprising the thiocarbonylthio group-containing intermediate, the second monomer, and a polymerization initiator.

One of the first monomer and the second monomer is a monomer that forms the monomer units of the block segment (A), and the other is a monomer that forms the monomer units of the block segment (B), and it is preferable that the first monomer is a monomer that forms the monomer units of the block segment (B) and the other is a monomer that forms the monomer units of the block segment (A), since the desired temperature-responsive block copolymer is more easily obtained.

The amount of the RAFT agent used per 1 mol of the first monomer may be, for example, 0.001 mol or more, or 0.005 mol or more, and may be 0.050 mol or less, or 0.020 mol or less.

The amount of the polymerization initiator used per 1 mol of the first monomer may be, for example, 0.0001 mol or more, or 0.0005 mol or more, and may be 0.0050 mol or less, or 0.0020 mol or less.

The reaction temperature in the polymerization step (1) may be, for example, 30°C or higher, and may be 120°C or lower. The time for maintaining the reaction temperature may be, for example, 1 hour or more, and may be 48 hours or less.

The thiocarbonylthio group-containing intermediate obtained in the polymerization step (1) comprises the block segment (A) or (B), and a terminal group bonded to the block segment (A) or (B) and containing a thiocarbonylthio group.

The amount of the second monomer used in the polymerization step (2) may be 100 mol or more, 150 mol or more, or 250 mol or more, and may be 400 mol or less, or 350 mol or less, per 1 mol of the thiocarbonylthio group-containing intermediate.

The amount of the polymerization initiator used in the polymerization step (2) may be 0.0001 mol or more, and may be 0.0010 mol or less, per 1 mol of the second monomer used. The amount of the polymerization initiator used in the polymerization step (2) may be 0.05 mol or more, and may be 0.20 mol or less, per 1 mol of the thiocarbonylthio group-containing intermediate.

The reaction temperature in the polymerization step (2) may be, for example, 30°C or higher, and may be 120°C or lower. The time for maintaining the reaction temperature may be, for example, 1 hour or more, and may be 48 hours or less.

The reactions in the polymerization steps (1) and (2) may be carried out in the presence of an organic solvent. Examples of the organic solvent include 1,4-dioxane, 1,2-dimethoxyethane, tert-butyl alcohol, tetrahydrofuran, N,N-dimethylformamide, ethanol, and 2-propanol. The reactions in the polymerization steps (1) and (2) may be carried out under a nitrogen or argon atmosphere.

The modification step may be, for example, a step of adjusting the amount of change in the bubble contact angle of the temperature-responsive block copolymer to 5.0° or more by introducing a polar group into the terminal of the thiocarbonylthio group-containing block copolymer.

The method for introducing a polar group into the terminal of the thiocarbonylthio group-containing block copolymer may be, for example, a reaction between the thiocarbonylthio group-containing block copolymer and a radical generator having a polar group.

As the radical generator, an azo-based or peroxide-based radical generator can be suitably used. Examples of the radical generator include azobisisobutyronitrile, 2,2'-azobis[2-methyl-N-(2-hydroxyethyl)propionamide], 4,4'-azobis-(4-cyanovaleric acid), dimethyl 2,2'-azobis-(2-methylpropionate), 2,2'-azobis[N-(2-carboxyethyl)-2-methylpropionamidine], 2,2'-azobis[2-(2-imidazolin-2-yl)propane], 2,2'-azobis(2-methylpropionamidine) dihydrochloride, 2,2'-azobis(N-butyl-2-methylpropionamide), 1,1'-azobis(cyclohexanecarbonitrile), dimethyl 1,1'-azobis(1-cyclohexanecarboxylate), 2,2'-azobis(2-methylbutyronitrile), 2,2'-azobis(4-methoxy-2,4-dimethylvaleronitrile), 2,2'-azobis(2,4-dimethylvaleronitrile), 2,2'-azobis(2-methylbutyronitrile), 1,1'-azobis(1-acetoxy-1-phenylethane), di-tert-butyl peroxide, tert-butyl hydroperoxide, hydrogen peroxide, potassium peroxodisulfate, benzoyl peroxide, cumene hydroperoxide, dicumyl peroxide, diisobutyl peroxide, di(3,5,5-trimethylhexanoyl) peroxide, disuccinic acid peroxide, diisopropyl peroxydicarbonate, dipropyl peroxydicarbonate, di-2-ethylhexyl peroxydicarbonate, and di-sec-butyl peroxydicarbonate. Among these, examples include azobisisobutyronitrile, 2,2'-azobis[2-methyl-N-(2-hydroxyethyl)propionamide], 4,4'-azobis-(4-cyanovaleric acid), and 2,2'-azobis-(2-methylpropionic acid). The radical generator may contain at least one selected from the group consisting of azobisisobutyronitrile, 2,2'-azobis[2-methyl-N-(2-hydroxyethyl)propionamide], 4,4'-azobis-(4-cyanovaleric acid), and 2,2'-azobis-(2-methylpropionic acid).

The amount of the radical generator used may be an excess amount relative to the thiocarbonylthio group-containing block copolymer. The amount of the radical generator used may be 5 mol or more, or 10 mol or more, and may be 100 mol or less, or 80 mol or less, per 1 mol of the thiocarbonylthio group-containing block copolymer.

By using the radical generators described above, a polar group can be efficiently introduced due to their decomposition temperature, solubility in the reaction solvent, and the low likelihood of side reactions occurring. When an azo-based or peroxide-based radical generator is used as the radical generator, a reaction mixture containing the thiocarbonylthio group-containing block copolymer and the radical generator can be reacted under conditions of, for example, 30 to 120°C for 1 to 48 hours.

The method for introducing a mercapto group as a polar group into the terminal of the thiocarbonylthio group-containing block copolymer may be a method of converting the terminal group containing the thiocarbonylthio group into a mercapto group (-SH) by hydrolysis.

The hydrolysis may be alkaline hydrolysis carried out under basic conditions. The alkaline hydrolysis can be carried out by reacting the thiocarbonylthio group-containing block copolymer in the presence of a base such as ethylamine, propylamine, butylamine, hexylamine, octylamine, benzylamine, ethylenediamine, hydrazine, piperidine, and ethanolamine.

The concentration of the base in the reaction solution may be 0.01 M or more, or 0.05 M or more, and may be 1 M or less, or 0.5 M or less.

When hydrolysis is carried out using a base, a reaction mixture containing the thiocarbonylthio group-containing block copolymer and the base can be reacted under conditions of, for example, 5 to 50°C for 10 to 180 minutes.

As one embodiment of the present invention, a method for producing a temperature-responsive block copolymer in which a polar group is introduced into a terminal of the block segment (A) or (B) is provided. The method includes a step of reacting the thiocarbonylthio group-containing block copolymer with a radical generator having a polar group to convert the terminal containing the thiocarbonylthio group into a terminal containing the polar group, thereby obtaining the temperature-responsive block copolymer. The step can be carried out by applying the aspects described as the method for introducing a polar group into a terminal.

As one embodiment of the present invention, a method for producing a temperature-responsive block copolymer in which a mercapto group is introduced into a terminal of the block segment (A) or (B) is provided. The method includes a step of hydrolyzing the thiocarbonylthio group-containing block copolymer to convert the terminal containing the thiocarbonylthio group into a mercapto group, thereby obtaining the temperature-responsive block copolymer. The step can be carried out by applying the aspects described as the method for introducing a mercapto group into a terminal.

### [Coating Agent]

The coating agent according to the present embodiment contains the temperature-responsive block copolymer and a solvent. The coating agent may further contain an additive as necessary. By using the coating agent, temperature responsiveness can be imparted to the surface of the cell culture substrate by a simple method of dropping the coating agent onto the surface of the cell culture substrate and drying it.

Examples of the solvent include water, an organic solvent, or a mixture thereof. Examples of the organic solvent include alcohols such as methanol, ethanol, 1-propanol, 2-propanol, and 1-butanol; and acetonitrile, formamide, N,N-dimethylformamide, dimethyl sulfoxide, tetrahydrofuran, 1,4-dioxane, and methyl ethyl ketone.

The content of the temperature-responsive block copolymer in the coating agent may be 0.01 to 50% by mass, 0.05 to 20% by mass, 0.1 to 10% by mass, or 0.5 to 5% by mass, based on the total mass of the coating agent.

### [Cell Culture Substrate]

The cell culture substrate according to the present embodiment includes a substrate and a coating layer covering at least a part of the surface of the substrate. The coating layer contains the temperature-responsive block copolymer described above. The amount of change in the bubble contact angle on the surface of the coating layer, as measured under respective conditions in water at 21°C and in water at 37°C, is 5.0° or more. The description given for the temperature-responsive block copolymer can be applied to the amount of change in the bubble contact angle on the surface of the coating layer.

The cell culture substrate may include a region having cell adhesiveness and temperature-responsive detachability, and may include a region having cell adhesiveness and temperature-responsive detachability, and a region of non-cell adhesiveness.

The region having cell adhesiveness and temperature-responsive detachability may be a region further having cell proliferativity. At least a part of the region having cell adhesiveness and temperature-responsive detachability is provided with a coating layer containing the temperature-responsive block copolymer.

The material of the substrate is not particularly limited, and not only substances commonly used for cell culture such as glass and polystyrene, but also substances that can generally be shaped, such as polymer compounds including polycarbonate, polyethylene terephthalate, polyvinylidene fluoride, polytetrafluoroethylene, polyethylene, polypropylene, and polymethyl methacrylate, as well as ceramics, metals, and the like can be used. From the viewpoint of ease of culture operations, the material of the substrate preferably contains at least one selected from the group consisting of glass, polystyrene, polycarbonate, polyethylene terephthalate, polyethylene, and polypropylene, more preferably contains at least one selected from the group consisting of glass, polystyrene, polycarbonate, polyethylene terephthalate, and polyethylene, and particularly preferably contains at least one selected from the group consisting of polystyrene, polycarbonate, polyethylene terephthalate, and polyethylene, since these are suitable for enhancing flexibility. The substrate may be polystyrene, since it can be more easily molded.

The substrate is preferably polycarbonate, since it is easy to form into a thin film and cell detachability is further improved when the temperature-responsive block copolymer is used.

The shape of the substrate is not particularly limited, and may be a planar shape such as a plate or a film, or may be a fiber, a porous particle, a porous membrane, or a hollow fiber. The substrate may also be a container commonly used for cell culture and the like (a cell culture dish such as a Petri dish, a flask, a plate, a bag, and the like.). From the viewpoint of ease of culture operations, the substrate is preferably a planar shape such as a plate or a film, or a flat porous membrane. From the viewpoint of being suitable for large-scale culture, the substrate is preferably a plate, a film, a flask, or a bag.

At least a part of the surface of the substrate may be coated with a hydrophilic polymer. That is, at least a part of the surface of the substrate may be provided with a layer containing a hydrophilic polymer. On the surface of the substrate, at least the region of non-cell adhesiveness may contain a hydrophilic polymer.

The hydrophilic polymer may be a hydrophilic polymer containing a phosphorylcholine group or a hydroxyl group. Since the hydrophilic polymer contains a phosphorylcholine group or a hydroxyl group, it is possible to make the region coated with the hydrophilic polymer a region to which cells do not adhere. In addition, since such a hydrophilic polymer does not need to be completely decomposed and removed, the region can also be made into a region having cell adhesiveness by a modification step. Examples of commercially available hydrophilic polymers include Lipidure (registered trademark) CM5206 (manufactured by NOF Corporation), Lipidure (registered trademark) CM2001 (manufactured by NOF Corporation), and BIOSURFINE (registered trademark)-AWP (manufactured by Toyo Gosei Co., Ltd.).

The hydrophilic polymer preferably contains a compound represented by the following General Formula (1), a compound represented by the following General Formula (2), or a compound represented by the following General Formula (3). 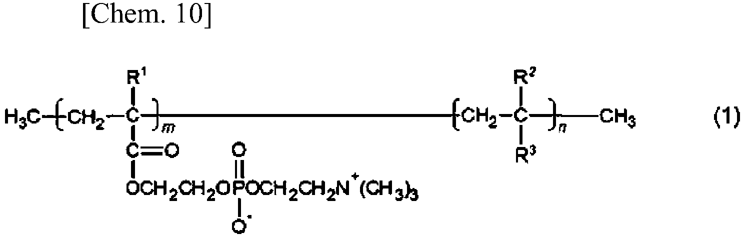 [In General Formula (1), R¹ and R² each independently represent a hydrogen atom or a methyl group, R³ represents a hydrogen atom or any organic group, and m and n each independently represent a positive integer.] 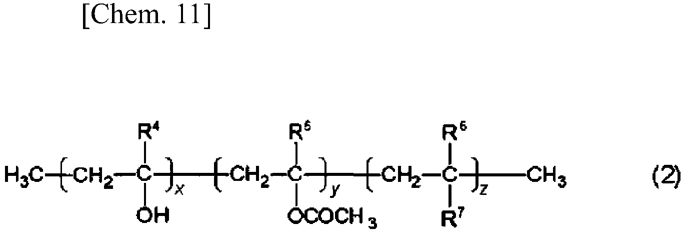 [In General Formula (2), R⁴, R⁵, and R⁶ each independently represent a hydrogen atom or a methyl group, R⁷ represents a hydrogen atom or any organic group, and x, y, and z each independently represent a positive integer.] 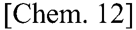 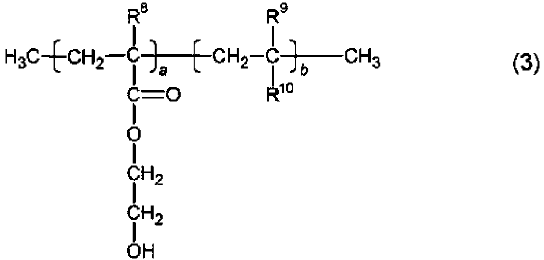 [In General Formula (3), R⁸R and ⁹ each independently represent a hydrogen atom or a methyl group, R¹⁰ represents a hydrogen atom or any organic group, and a and b each independently represent a positive integer.]

When the hydrophilic polymer contains the compound represented by General Formula (1), the compound represented by General Formula (2), or the compound represented by General Formula (3), cells can more easily adhere and proliferate, and it is suitable for forming cell aggregates of uniform shape in recessed portions. In General Formula (1), General Formula (2), or General Formula (3), R³, R⁷, and R¹⁰ are each preferably a hydrophobic group or a functional group having reactivity to active energy rays (for example, UV, electron beam, etc.), since they are suitable for immobilizing the hydrophilic polymer on the substrate. As the hydrophobic group, linear or cyclic alkyl groups such as a methyl group, an ethyl group, a propyl group, a butyl group, and a cyclohexyl group can be suitably used. Examples of the functional group having reactivity to active energy rays (for example, UV, electron beam, etc.) include an azide group, an acrylate group, a methacrylate group, a vinyl group, and an epoxy group, and an azide group can be suitably used.

The layer thickness of the layer containing the hydrophilic polymer may be, for example, 10 nm or more, 20 nm or more, 30 nm or more, or 40 nm or more, and may be 50 nm or more, 60 nm or more, or 70 nm or more, since it is suitable for suppressing adhesion of cells to regions other than the region having cell adhesiveness and temperature-responsive detachability. The layer thickness of the layer containing the hydrophilic polymer may be 1500 nm or less, and may be 1000 nm or less, 500 nm or less, 200 nm or less, 150 nm or less, 100 nm or less, 90 nm or less, or 80 nm or less, since it is suitable for increasing the cell survival rate of cell aggregates by collecting cells in the region having cell adhesiveness and temperature-responsive detachability through cell migration.

The "layer thickness" of the layer containing the hydrophilic polymer refers to the length in the out-of-plane direction from the interface between the substrate and the layer containing the hydrophilic polymer to the interface (excluding recessed portions) on the side opposite to the substrate of the layer containing the hydrophilic polymer. In the range where the layer thickness exceeds 10 nm, it can be calculated by measuring an ultrathin section of the cell culture substrate prepared by a microtome using a transmission electron microscope to obtain a cross-sectional image, measuring the distance at 10 randomly selected points, and averaging the values. In the range where the layer thickness is 10 nm or less, it can be measured using an ellipsometer.

The thickness of the substrate is preferably 0.01 mm or more and 0.5 mm or less, 0.05 mm or more and 0.4 mm or less, 0.1 mm or more and 0.3 mm or less, or 0.15 mm or more and 0.25 mm or less. When the thickness of the substrate is within this range, the fluorescence image of cells observed at high magnification becomes clearer.

Commercially available products can be used as the substrate. Examples of the substrate include commercially available culture substrates (for example, trade name: PrimeSurface (registered trademark) manufactured by Sumitomo Bakelite Co., Ltd., and trade name: EZ-BindShut (registered trademark) manufactured by AGC Techno Glass Co., Ltd.). As the substrate, commercially available substrates pre-coated with a hydrophilic polymer, such as PrimeSurface (registered trademark) (manufactured by Sumitomo Bakelite Co., Ltd.), EZ-BindShut (registered trademark) (manufactured by AGC Techno Glass Co., Ltd.), and EZ-BindShutII (registered trademark) (manufactured by AGC Techno Glass Co., Ltd.), can be suitably used.

At least a part of the surface of the substrate may be subjected to a surface modification treatment. The region of the substrate where the coating layer is to be provided (the portion to be made into the region having cell adhesiveness and temperature-responsive detachability) may be subjected to a surface modification treatment. Examples of the surface modification treatment include plasma treatment, corona treatment, and UV treatment. The surface modification treatments such as plasma treatment, corona treatment, and UV treatment can be carried out by applying conditions commonly used when treating the surface of a cell culture substrate so as to have cell adhesiveness and cell proliferativity.

The layer thickness of the coating layer may be 1000 nm or less, 500 nm or less, 200 nm or less, 100 nm or less, 80 nm or less, 60 nm or less, 50 nm or less, 40 nm or less, or 35 nm or less, since cell adhesiveness is further improved. The layer thickness of the coating layer may be 5 nm or more, more than 5 nm, 10 nm or more, 15 nm or more, 20 nm or more, or 25 nm or more, since cell detachability is further improved. The layer thickness of the coating layer may be 5 to 1000 nm, 10 to 500 nm, 10 to 200 nm, 10 to 100 nm, 10 to 50 nm, 15 to 200 nm, 15 to 100 nm, 15 to 50 nm, 20 to 200 nm, 20 to 100 nm, 20 to 50 nm, 25 to 200 nm, 25 to 100 nm, or 25 to 50 nm, since cell detachability is further improved.

The layer thickness of the coating layer is measured by a scanning probe microscope (AFM) apparatus (for example, SPM-9600 manufactured by Shimadzu Corporation). The layer thickness of the coating layer is measured by making a scratch on the surface with tweezers and measuring the depth of the scratch using a cantilever BL-AC40TS-C2.

The coating layer may have a phase-separated structure, or a surface structure derived from phase separation, at a culture temperature. The culture temperature may be, for example, 30 to 42°C, 32 to 40°C, 36 to 38°C, or 37°C. The method for forming the surface structure by phase separation is not particularly limited, and examples thereof include a method of causing phase separation when the block copolymer is applied to the substrate, and a method of applying a block copolymer that undergoes phase separation at the culture temperature.

The cell culture substrate may be sterilized. The sterilization method is not particularly limited, and high-pressure steam sterilization, UV sterilization, gamma-ray sterilization, ethylene oxide gas sterilization, and the like can be used. From the viewpoint of suppressing denaturation of the block copolymer, high-pressure steam sterilization, UV sterilization, and ethylene oxide gas sterilization are preferred. From the viewpoint of suppressing deformation of the substrate, UV sterilization or ethylene oxide gas sterilization is preferred. From the viewpoint of excellent mass productivity, ethylene oxide gas sterilization is preferred.

The cell culture substrate can be obtained by a method including a step of forming a coating layer containing the temperature-responsive block copolymer described above on the substrate. By including the step of forming the coating layer on the substrate, the cell detachability of the cell culture substrate is improved. The method for forming the coating layer is not particularly limited, and various commonly known methods such as coating, brush coating, dip coating, spin coating, bar coating, flow coating, spray coating, roll coating, air knife coating, blade coating, gravure coating, micro gravure coating, and slot die coating can be used.

A cell culture substrate including a region having cell adhesiveness and temperature-responsive detachability, and a region of non-cell adhesiveness, can be produced by a method including a step of subjecting a part of the surface of a substrate whose surface is coated with a hydrophilic polymer (the portion to be made into the region having cell adhesiveness and temperature-responsive detachability) to a surface modification treatment, and a step of forming a coating layer comprising the temperature-responsive block copolymer on the substrate of which a part has been surface-modified. As a method for subjecting a part of the surface of the substrate to a surface modification treatment, for example, a method including placing a mask (for example, a metal mask) having holes of a predetermined shape (for example, a circular shape) on the substrate, and subjecting the substrate on which the mask is placed to a surface modification treatment can be mentioned. According to this method, the region subjected to the surface modification treatment (the region of the holes of the mask) can be patterned as a region having cell adhesiveness, and the region not subjected to the surface modification treatment (the mask region) can be patterned as a region of non-cell adhesiveness.

### [Method for Producing Cells]

The method for producing cells includes a culture step of culturing cells on the cell culture substrate described above under a temperature condition equal to or higher than the temperature at which the degree of hydrophilicity and hydrophobicity of the temperature-responsive block copolymer changes (hereinafter referred to as "response temperature"), and a detachment step of detaching cells from the cell culture substrate after cooling the cell culture substrate to below the response temperature. The method for producing cells may further include a seeding step of seeding cells onto the cell culture substrate before the culture step.

In the seeding step, cells are seeded onto the cell culture substrate. After seeding, the cells adhere to the cell substrate. The seeding of cells is carried out by, for example, bringing a medium in which cells are dispersed (hereinafter also referred to as "cell suspension") into contact with the cell culture substrate by applying the cell suspension onto the cell culture substrate or injecting it into the culture substrate.

The cells are not particularly limited, and examples thereof include various established cell lines such as Chinese hamster ovary-derived CHO cells, mouse connective tissue L929, human embryonic kidney-derived HEK293 cells, and human cervical cancer-derived HeLa cells, as well as epithelial cells and endothelial cells constituting various tissues and organs in the living body, skeletal muscle cells, smooth muscle cells, and cardiomyocytes exhibiting contractility, neuron cells and glial cells constituting the nervous system, fibroblasts, hepatocytes and non-parenchymal liver cells involved in metabolism in the living body, adipocytes, stem cells present in various tissues such as mesenchymal stem cells, bone marrow cells, and Muse cells as cells having differentiation potential, and further stem cells having pluripotency (pluripotent stem cells) such as ES cells and iPS cells, and cells induced to differentiate therefrom. From the viewpoint of cell proliferativity and detachability on the cell culture substrate, stem cells or pluripotent stem cells are preferred, mesenchymal stem cells or pluripotent stem cells are more preferred, pluripotent stem cells are still more preferred, and iPS cells are most preferred.

In the culture step, cells are cultured on the cell culture substrate described above under a temperature condition equal to or higher than the response temperature. The culture of cells is carried out in a medium. The temperature of the medium when culturing cells is equal to or higher than the response temperature, and is preferably 30 to 42°C, 32 to 40°C, or 36 to 38°C.

The medium can be appropriately selected according to the type of cells and the like. The medium may contain additives according to the type of cells. When the cells are stem cells, the medium may contain a factor for maintaining the undifferentiated state of the stem cells. Examples of the factor for maintaining the undifferentiated state of stem cells include insulin, transferrin, selenium, ascorbic acid, sodium bicarbonate, basic fibroblast growth factor, transforming growth factor β (TGFβ), CCL2, activin, and 2-mercaptomethanol.

Examples of the medium include DMEM (manufactured by Sigma-Aldrich Co. LLC), Ham's F12 (manufactured by Sigma-Aldrich Co. LLC), D-MEM/Ham's F12 (manufactured by Sigma-Aldrich Co. LLC), Primate ES Cell Medium (manufactured by REPROCELL Inc.), StemFit AK02N (manufactured by Ajinomoto Co., Inc.), StemFit AK03 (manufactured by Ajinomoto Co., Inc.), mTeSR1 (manufactured by STEMCELL TECHNOLOGIES), TeSR-E8 (manufactured by STEMCELL TECHNOLOGIES), ReproNaive (manufactured by REPROCELL Inc.), ReproXF (manufactured by REPROCELL Inc.), ReproFF (manufactured by REPROCELL Inc.), ReproFF2 (manufactured by REPROCELL Inc.), NutriStem (manufactured by Biological Industries), iSTEM (manufactured by Takara Bio Inc.), GS2-M (manufactured by Takara Bio Inc.), and hPSC Growth Medium DXF (manufactured by PromoCell GmbH).

The cell density at the start of culture is not particularly limited, and may be 1.0×10² to 1.0×10⁶ cells/cm², 1.0×10³ to 5.0×10⁵ cells/cm², or 1.0×10⁴ to 1.0×10⁵ cells/cm².

The medium may contain, for example, a biologically derived substance. The biologically derived substance may be a natural product, may be artificially synthesized by genetic recombination technology or the like, may be a fragment cleaved by a restriction enzyme or the like, or may be a synthetic protein or synthetic peptide based on these biologically derived substances. A specific example of the biologically derived substance is iMatrix-511 (manufactured by Nippi, Inc.). The concentration of the biologically derived substance in the medium may be, for example, 1.0 to 10 µL/mL or 2.0 to 5 µL/mL, based on the total amount of the medium.

The medium may further contain a Rho-associated kinase inhibitor. Examples of the Rho-associated kinase inhibitor include (R)-(+)-trans-N-(4-pyridyl)-4-(1-aminoethyl)-cyclohexanecarboxamide·2HCl·H₂O (Y-27632, manufactured by Fujifilm Wako Pure Chemical Corporation) and 1-(5-Isoquinolinesulfonyl)homopiperazine Hydrochloride (HA1077, manufactured by Fujifilm Wako Pure Chemical Corporation). The concentration of the Rho-associated kinase inhibitor may be within a range effective for maintaining the survival of human stem cells and within a range that does not affect the undifferentiated state of human stem cells, and may be, for example, 1 µM to 50 µM, 3 µM to 20 µM, 5 µM to 15 µM, or 8 µM to 12 µM, based on the total amount of the medium.

The culture period can be appropriately set according to the type of cells and the like. When culturing stem cells, it is preferable to culture the cells by the following method. The first medium exchange is performed 22 to 26 hours after the initial start of cell culture. The second medium exchange is performed 48 to 72 hours after the start of the first culture. After the second medium exchange, the medium is exchanged every 24 to 48 hours. The cells proliferate by culture and form cell aggregates called colonies. The culture may be continued until the size of the colonies reaches approximately 1 mm.

In the detachment step, cells are detached from the cell culture substrate after cooling the cell culture substrate to below the response temperature. By cooling the cell culture substrate to below the response temperature, the degree of hydrophilicity and hydrophobicity of the coating layer on the cell culture substrate changes, making it easier for cells to detach from the cell culture substrate. The cooling of the cell culture substrate can be carried out by, for example, a method of removing the medium after the culture step and then adding a liquid at a temperature below the response temperature. The liquid temperature of the liquid at a temperature below the response temperature may be, for example, 0 to 20°C, 1 to 15°C, 2 to 10°C, or 3 to 5°C. The time for bringing the cell culture substrate into contact with the liquid at a temperature below the response temperature may be, for example, 120 minutes or less, 100 minutes or less, 60 minutes or less, or 30 minutes or less, and may be 5 minutes or more, 10 minutes or more, or 15 minutes or more.

The detachment of cells can be carried out by applying an external stimulus to the cell culture substrate. The external stimulus may be, for example, pipetting, tapping, or stirring with a shaker. Pipetting refers to an operation of repeatedly aspirating and discharging the liquid contained in the cell culture solution using an instrument such as a Pipetman. Tapping refers to an operation of applying vibration to the culture environment such as by tapping the cell culture substrate. Stirring with a shaker refers to an operation of applying an external stimulus with a shaker, and the stirring method is not particularly limited. Stirring with a shaker may be, for example, an operation of applying vibration (rotational vibration) by rotating the cell culture substrate in a circular motion. The shaker may be a device capable of rotating the cell culture substrate in a horizontal circle, and a commercially available microplate shaker or the like can be used.

### Examples

Hereinafter, the present invention will be described in detail with reference to embodiments for carrying out the present invention, but these are examples for explaining the present invention and are not intended to limit the present invention to the following contents. The present invention can also be carried out with appropriate modifications within the scope of the gist of the present invention. Unless otherwise specified, commercially available reagents were used.

### <Composition of Polymer>

The composition of the polymer was determined by proton nuclear magnetic resonance spectroscopy (¹H-NMR) spectral analysis using a nuclear magnetic resonance measurement apparatus (manufactured by JEOL Ltd., trade name: JNM-GSX400), or by carbon nuclear magnetic resonance spectroscopy (¹³C-NMR) spectral analysis using a nuclear magnetic resonance measurement apparatus (manufactured by Bruker, trade name: AVANCE III HD500).

### <Molecular Weight and Molecular Weight Distribution of Polymer>

The weight-average molecular weight (Mw), number-average molecular weight (Mn), and molecular weight distribution (Mw/Mn) were measured by gel permeation chromatography (GPC). The GPC apparatus used was HLC-8320GPC manufactured by Tosoh Corporation, two columns of TSKgel Super AWM-H manufactured by Tosoh Corporation were used, the column temperature was set to 40°C, and N,N-dimethylformamide containing 10 mM lithium bromide was used as the eluent. The measurement sample was prepared at 1.0 mg/mL for measurement. A calibration curve for molecular weight was prepared using polymethyl methacrylate of known molecular weight (manufactured by Polymer Laboratories Ltd.).

### <Degree of Polymerization of Block Segment (A)>

The degree of polymerization of the block segment (A) was calculated based on the following formula. 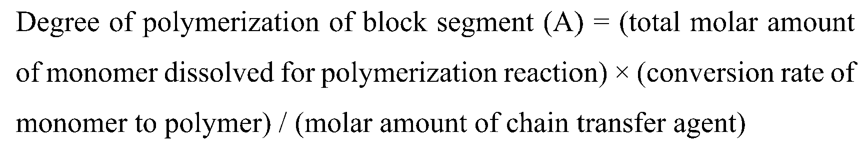 Degree of polymerization of block segment (A) = (total molar amount of monomer dissolved for polymerization reaction) × (conversion rate of monomer to polymer) / (molar amount of chain transfer agent)

### <Layer Thickness of Coating Layer>

The layer thickness of the coating layer containing the block copolymer provided on the substrate was measured by an AFM apparatus (SPM-9600 manufactured by Shimadzu Corporation). A cantilever BL-AC40TS-C2 was used, a scratch was made on the surface with tweezers, and the layer thickness of the coating layer was measured by measuring the depth of the scratch.

### Example 1

### [Synthesis of Block Copolymer]

0.10 g (0.25 mmol) of 4-cyano-4-[(dodecylsulfanylthiocarbonyl)sulfanyl]pentanoic acid, 2.99 g (21 mmol) of n-butyl methacrylate, and 8 mg (0.05 mmol) of azobisisobutyronitrile were added to a test tube and dissolved in 18 mL of 1,4-dioxane. After degassing by nitrogen bubbling for 30 minutes, the mixture was reacted at 70°C for 17 hours to obtain an n-butyl methacrylate polymer solution. The conversion rate of n-butyl methacrylate at this stage was 80%.

21 mL (0.25 mmol) of the n-butyl methacrylate polymer solution, 7.92 g (70 mmol) of N-isopropylacrylamide, and 8 mg (0.03 mmol) of azobisisobutyronitrile were added to a 100 mL eggplant-shaped flask and dissolved in 2 mL of 1,4-dioxane. After degassing by nitrogen bubbling for 30 minutes, the mixture was reacted at 70°C for 3 hours. The conversion rate of N-isopropylacrylamide at this stage was 85%. After completion of the reaction, the reaction solvent was diluted with 70 mL of acetone, poured into 3 L of hexane, and the precipitated solid was collected and dried under reduced pressure to prepare block copolymer A. Block copolymer A is a block copolymer containing a block segment (A) containing N-isopropylacrylamide as a monomer unit and a block segment (B) containing n-butyl methacrylate as a monomer unit.

1 g of block copolymer A and 0.32 g (2.0 mmol) of azobisisobutyronitrile were added to a test tube and dissolved in 20 mL of 1,4-dioxane. After degassing by nitrogen bubbling for 30 minutes, the mixture was reacted at 80°C for 3 hours. After completion of the reaction, the reaction solution was poured into 1 L of hexane, the precipitated solid was collected and dried under reduced pressure, then redissolved in acetone, poured into 1 L of pure water, and the precipitated gel-like product was collected and freeze-dried. Thus, the block copolymer of Example 1 was obtained.

The block copolymer of Example 1 is a copolymer in which a cyano group is introduced into the terminal on the block segment (A) side. The degree of polymerization of the block segment (A) in the block copolymer of Example 1 was 238. The Mn×10⁻³ of the block copolymer of Example 1 was 38.4, and the Mw/Mn was 1.15.

### [Measurement of Bubble Contact Angle]

The block copolymer of Example 1 was diluted with ethanol to prepare a 0.8% by mass solution. 100 µL of the prepared 0.8% by mass solution was dropped onto a polycarbonate film (manufactured by Teijin Limited, trade name: Panlite 180) and spin-coated at a rotation speed of 2000 rpm for 60 seconds to form a film of the block copolymer of Example 1. The polycarbonate film coated with the film of the block copolymer of Example 1 was immersed in water at 21°C or 37°C for 10 minutes, and the bubble contact angle (°) was measured using a contact angle meter DM300 manufactured by Kyowa Interface Science Co., Ltd. in water using a bubble of 1 µL. In the measurement of the bubble contact angle, the film thickness of the block copolymer was 40 nm.

The bubble contact angle measured using the block copolymer of Example 1 was 145.1° at 21°C and 156.2° at 37°C, and the amount of change in the bubble contact angle was 11.1°.

### [Preparation of Cell Culture Substrate]

A 35-mm diameter dish having a surface coated with a hydrophilic polymer (manufactured by Sumitomo Bakelite Co., Ltd., trade name: PrimeSurface (registered trademark)) was covered with a metal mask (manufactured by Mitani Micronics Co., Ltd.) having a plurality of circular holes with a diameter of 0.5 mm, and plasma treatment (under a gas pressure of 20 Pa, a conduction current of 20 mA, and an irradiation time of 60 seconds) was performed from above the metal mask using a plasma irradiation apparatus (manufactured by Vacuum Device Inc., trade name: Plasma Ion Bombarder PIB-20) to prepare a cell culture substrate having a region of cell adhesiveness and cell proliferativity. 50 µL of a 0.6% by mass ethanol solution of the block copolymer of Example 1 was dropped onto this cell culture substrate and spin-coated at 2000 rpm for 60 seconds to form a coating layer containing the block copolymer of Example 1 on the substrate, thereby preparing the cell culture substrate of Example 1. The layer thickness of the coating layer in the cell culture substrate of Example 1 was 30 nm.

### [Cell Culture and Evaluation of Cell Detachability]

0.2 mL/cm² of medium StemFit AK02N (manufactured by Ajinomoto Co., Inc.) was added to two substrates coated with the block copolymer of Example 1, and human iPS cells of 201B7 strain were further added at 15,000 cells/cm² and iMatrix-511 solution (manufactured by Nippi, Inc.) was added at a concentration of 2.5 µL/mL. The cells were cultured at 37°C in an environment with a CO₂ concentration of 5%. In addition, Y-27632 (manufactured by Fujifilm Wako Pure Chemical Corporation) (concentration: 10 µM) was added to the medium until 24 hours after cell seeding, and at 24 hours and 96 hours after seeding, the state of the cells was observed with a phase contrast microscope and the medium was replaced with fresh medium. At 144 hours after cell seeding, it was confirmed that cell aggregates were formed in a pattern on the bottom surface of the culture substrate.

After the medium was removed from one of the two culture substrates, the substrate was washed with phosphate-buffered saline (PBS(-)), methanol (manufactured by Fujifilm Wako Pure Chemical Corporation) was added, and the cell aggregates were fixed by allowing the substrate to stand for 30 minutes. Thereafter, the methanol was removed, the substrate was washed again with PBS(-), Giemsa staining solution was added, and staining was performed at room temperature for 30 minutes. After the staining solution was removed and the substrate was washed with PBS(-), the substrate was dried, the entire bottom surface of the substrate was photographed with a fluorescence microscope (manufactured by Keyence Corporation, trade name: BZ-X800), and the number of stained cell aggregates was counted using an image analysis application (Image-Pro (registered trademark) manufactured by Media Cybernetics).

After the medium was removed from the other culture substrate, PBS(-) at 4°C was added thereto, the substrate was allowed to stand for 20 minutes, and the side surface of the substrate was tapped to apply vibration to detach the cell aggregates. After the PBS was removed together with the detached cell aggregates, Giemsa staining was performed by the method described above, and the number of cell aggregates that had not detached was counted.

From the respective numbers of the cell aggregate counted, the proportion of detached cell aggregates among the cell aggregates formed on the culture substrate was calculated to be 79.4%.

### Example 2

### [Synthesis of Block Copolymer]

Block copolymer A containing a block segment (A) containing N-isopropylacrylamide as a monomer unit and a block segment (B) containing n-butyl methacrylate as a monomer unit was synthesized in the same manner as described in [Synthesis of Block Copolymer] of Example 1.

1 g of block copolymer A and 0.43 g (2.0 mmol) of 2,2'-azobis[2-methyl-N-(2-hydroxyethyl)propionamide] were added to a test tube and dissolved in 20 mL of 1,4-dioxane. After the mixture was degassed by nitrogen bubbling for 30 minutes, the mixture was reacted at 90°C for 14 hours. After completion of the reaction, the reaction solution was poured into 1 L of hexane, and the precipitated solid was collected and dried under reduced pressure, then redissolved in acetone, and poured into 1 L of pure water, and the precipitated gel-like product was collected and freeze-dried. This gave the block copolymer of Example 2.

The block copolymer of Example 2 is a copolymer in which a hydroxyl group is introduced into the terminal on the block segment (A) side. The degree of polymerization of the block segment (A) in the block copolymer of Example 2 was 238. The Mn×10⁻³ of the block copolymer of Example 2 was 38.0, and the Mw/Mn was 1.18.

### [Measurement of Bubble Contact Angle]

A film coated with the block copolymer was prepared and the bubble contact angle was measured in the same manner as in Example 1 [Measurement of Bubble Contact Angle], except that the block copolymer of Example 2 was used. The bubble contact angle measured using the block copolymer of Example 2 was 155.0° at 21°C and 122.7° at 37°C, and the amount of change in the bubble contact angle was 32.3°.

### [Preparation of Cell Culture Substrate]

The cell culture substrate of Example 2 was prepared in the same manner as in Example 1 [Preparation of Cell Culture Substrate], except that the block copolymer of Example 2 was used.

### [Cell Culture and Evaluation of Cell Detachability]

Cell culture and evaluation of cell detachability were carried out in the same manner as in Example 1 [Cell Culture and Evaluation of Cell Detachability], except that the cell culture substrate of Example 2 was used. The proportion of detached cell aggregates among the cell aggregates formed on the prepared cell culture substrate was 85.0%.

### Example 3

### [Synthesis of Block Copolymer]

Block copolymer A comprising a block segment (A) containing N-isopropylacrylamide as a monomer unit and a block segment (B) containing n-butyl methacrylate as a monomer unit was synthesized in the same manner as described in [Synthesis of Block Copolymer] of Example 1.

1 g of block copolymer A and 0.42 g (2.0 mmol) of 4,4'-azobis-(4-cyanovaleric acid) were added to a test tube and dissolved in 20 mL of 1,4-dioxane. After degassing by nitrogen bubbling for 30 minutes, the mixture was reacted at 90°C for 14 hours. After completion of the reaction, the reaction solution was poured into 1 L of hexane, the precipitated solid was collected and dried under reduced pressure, then redissolved in acetone, poured into 1 L of pure water, and the precipitated gel-like product was collected and freeze-dried. This gave the block copolymer of Example 3.

The block copolymer of Example 3 is a copolymer in which a carboxyl group is introduced into the terminal on the block segment (A) side. The degree of polymerization of the block segment (A) in the block copolymer of Example 3 was 238. The Mn×10⁻³ of the block copolymer of Example 3 was 35.3, and the Mw/Mn was 1.18.

### [Measurement of Bubble Contact Angle]

A film coated with the block copolymer was prepared and the bubble contact angle was measured in the same manner as in Example 1 [Measurement of Bubble Contact Angle], except that the block copolymer of Example 3 was used. The bubble contact angle measured using the block copolymer of Example 3 was 159.3° at 21°C and 135.6° at 37°C, and the amount of change in the bubble contact angle was 23.7°.

### [Preparation of Cell Culture Substrate]

The cell culture substrate of Example 3 was prepared in the same manner as in Example 1 [Preparation of Cell Culture Substrate], except that the block copolymer of Example 3 was used.

### [Cell Culture and Evaluation of Cell Detachability]

Cell culture and evaluation of cell detachability were carried out in the same manner as in Example 1 [Cell Culture and Evaluation of Cell Detachability], except that the cell culture substrate of Example 3 was used. The proportion of detached cell aggregates among the cell aggregates formed on the prepared culture substrate was 88.4%.

### Example 4

### [Synthesis of Block Copolymer]

Block copolymer A containing a block segment (A) containing N-isopropylacrylamide as a monomer unit and a block segment (B) containing n-butyl methacrylate as a monomer unit was synthesized in the same manner as described in [Synthesis of Block Copolymer] of Example 1.

1 g of block copolymer A and 0.35 g (2.0 mmol) of 2,2'-azobis-(2-methylpropionic acid) were added to a test tube and dissolved in 20 mL of 1,4-dioxane. After degassing by nitrogen bubbling for 30 minutes, the mixture was reacted at 80°C for 3 hours. After completion of the reaction, the reaction solution was poured into 1 L of hexane, the precipitated solid was collected and dried under reduced pressure, then redissolved in acetone, poured into 1 L of pure water, and the precipitated gel-like product was collected and freeze-dried. This gave the block copolymer of Example 4.

The block copolymer of Example 4 is a copolymer in which an ester group (-C(=O)OCH₃) is introduced into the terminal on the block segment (A) side. The degree of polymerization of the block segment (A) in the block copolymer of Example 4 was 238. The Mn×10⁻³ of the block copolymer of Example 4 was 37.5, and the Mw/Mn was 1.16.

### [Measurement of Bubble Contact Angle]

A film coated with the block copolymer was prepared and the bubble contact angles were measured in the same manner as in Example 1 [Measurement of Bubble Contact Angle], except that the block copolymer of Example 4 were used. The bubble contact angle measured using the block copolymer of Example 4 was 160.1° at 21°C and 133.5° at 37°C, and the amount of change in the bubble contact angle was 26.6°.

### [Preparation of Cell Culture Substrate]

The cell culture substrate of Example 4 was prepared in the same manner as in Example 1 [Preparation of Cell Culture Substrate], except that the block copolymer of Example 4 was used.

### [Cell Culture and Evaluation of Cell Detachability]

Cell culture and evaluation of cell detachability were carried out in the same manner as in Example 1 [Cell Culture and Evaluation of Cell Detachability], except that the cell culture substrate of Example 4 was used. The proportion of detached cell aggregates among the cell aggregates formed on the prepared cell culture substrate was 89.6%.

### Example 5

### [Synthesis of Block Copolymer]

Block copolymer A containing a block segment (A) containing N-isopropylacrylamide as a monomer unit and a block segment (B) containing n-butyl methacrylate as a monomer unit was synthesized in the same manner as described in [Synthesis of Block Copolymer] of Example 1.

1 g of block copolymer A and 0.52 g (4.0 mmol) of octylamine were added to a test tube and dissolved in 20 mL of 1,4-dioxane, and then reacted at 25°C for 30 minutes. After completion of the reaction, the reaction solution was poured into 1 L of hexane, and the precipitated solid was collected and dried under reduced pressure, then redissolved in acetone and poured into 1 L of pure water, and the precipitated gel-like product was collected and freeze-dried. This gave the block copolymer of Example 5.

The block copolymer of Example 5 is a copolymer in which a mercapto group is introduced into the terminal on the block segment (A) side. The degree of polymerization of the block segment (A) in the block copolymer of Example 5 was 238. The Mn×10⁻³ of the block copolymer of Example 5 was 36.0, and the Mw/Mn was 1.25.

### [Measurement of Bubble Contact Angle]

A film coated with the block copolymer was prepared and the bubble contact angle was measured in the same manner as in Example 1 [Measurement of Bubble Contact Angle], except that the block copolymer of Example 5 was used. The bubble contact angle measured using the block copolymer of Example 5 was 151.2° at 21°C and 127.1° at 37°C, and the amount of change in the bubble contact angle was 24.1°.

### [Preparation of Cell Culture Substrate]

The cell culture substrate of Example 5 was prepared in the same manner as in Example 1 [Preparation of Cell Culture Substrate], except that the block copolymer of Example 5 was used.

### [Cell Culture and Evaluation of Cell Detachability]

Cell culture and evaluation of cell detachability were carried out in the same manner as in Example 1 [Cell Culture and Evaluation of Cell Detachability], except that the cell culture substrate of Example 5 was used. The proportion of detached cell aggregates among the cell aggregates formed on the prepared cell culture substrate was 80.3%.

### Comparative Example 1

### [Synthesis of Block Copolymer]

0.10 g (0.25 mmol) of 4-cyano-4-[(dodecylsulfanylthiocarbonyl)sulfanyl]pentanoic acid, 2.99 g (21 mmol) of n-butyl methacrylate, and 8 mg (0.05 mmol) of azobisisobutyronitrile were added to a test tube and dissolved in 18 mL of 1,4-dioxane. After degassing by nitrogen bubbling for 30 minutes, the mixture was reacted at 70°C for 17 hours to obtain an n-butyl methacrylate polymer solution. The conversion rate of n-butyl methacrylate at this stage was 80%.

21 mL (0.25 mmol) of the n-butyl methacrylate polymer solution, 7.92 g (70 mmol) of N-isopropylacrylamide, and 8 mg (0.03 mmol) of azobisisobutyronitrile were added to a 100 mL eggplant-shaped flask and dissolved in 2 mL of 1,4-dioxane. After degassing by nitrogen bubbling for 30 minutes, the mixture was reacted at 70°C for 3 hours. The conversion rate of N-isopropylacrylamide at this stage was 85%. After completion of the reaction, the reaction solvent was diluted with 70 mL of acetone, poured into 3 L of hexane, and the precipitated solid was collected and dried under reduced pressure. Then, 1 g of the obtained polymer was redissolved in 15 mL of acetone, poured into 1 L of pure water, and the precipitated gel-like product was collected and freeze-dried. This gave the block copolymer of Comparative Example 1.

The block copolymer of Comparative Example 1 is a terminal-unmodified block copolymer in which a structure derived from the RAFT agent remains at the terminal. The degree of polymerization of the block segment (A) in the block copolymer of Comparative Example 1 was 238. The Mn×10⁻³ of the block copolymer of Comparative Example 1 was 35.6, and the Mw/Mn was 1.17.

### [Measurement of Bubble Contact Angle]

A film coated with the block copolymer was prepared and the bubble contact angle was measured in the same manner as in Example 1 [Measurement of Bubble Contact Angle], except that the block copolymer of Comparative Example 1 was used. The bubble contact angle measured using the block copolymer of Comparative Example 1 was 155.5° at 20°C and 151.2° at 37°C, and the amount of change in the bubble contact angle was 4.3°.

### [Preparation of Cell Culture Substrate]

The cell culture substrate of Comparative Example 1 was prepared in the same manner as in Example 1 [Preparation of Cell Culture Substrate], except that the block copolymer of Comparative Example 1 was used.

### [Cell Culture and Evaluation of Cell Detachability]

Cell culture and evaluation of cell detachability were carried out in the same manner as in Example 1 [Cell Culture and Evaluation of Cell Detachability], except that the cell culture substrate of Comparative Example 1 was used. The proportion of detached cell aggregates among the cell aggregates formed on the prepared cell culture substrate was 44.2%.

### Comparative Example 2

### [Preparation of Cell Culture Substrate]

The cell culture substrate of Comparative Example 2 was prepared in the same manner as in Example 1 [Preparation of Substrate Coated with Terminal-Modified Block Copolymer], except that the step of coating the substrate with the block copolymer was omitted.

### [Cell Culture and Evaluation of Cell Detachability]

Cell culture and evaluation of cell detachability were carried out in the same manner as in Example 1 [Cell Culture and Evaluation of Cell Detachability], except that the cell culture substrate of Comparative Example 2 was used. The proportion of detached cell aggregates among the cell aggregates formed on the prepared culture substrate was 3.1%.

### Comparative Example 3

### [Measurement of Bubble Contact Angle]

The bubble contact angle of a polycarbonate film not coated with a block copolymer was measured in the same manner as in Example 1 [Measurement of Bubble Contact Angle]. The measured bubble contact angle was 131.7° at 21°C and 129.9° at 37°C, and the amount of change in the bubble contact angle was 1.8°.

### Example 6

### [Preparation of Cell Culture Substrate with Film-Formed Bottom Surface]

0.1 mL of an 80 wt% ethanol aqueous solution containing a hydrophilic polymer (manufactured by Toyo Gosei Co., Ltd., trade name: BIOSURFINE (registered trademark)-AWP) at a solid content concentration of 0.6 wt% was dropped onto the polycarbonate film and spin-coated (2000 rpm, 60 seconds), and then the film was allowed to stand under a high-pressure mercury lamp for 1 hour to perform UV irradiation to cure the hydrophilic polymer, thereby forming a layer containing the hydrophilic polymer (layer thickness: 75 nm). The film coated with this hydrophilic polymer was covered with the metal mask having a plurality of circular holes with a diameter of 0.5 mm, and plasma treatment (under a gas pressure of 20 Pa, a conduction current of 20 mA, and an irradiation time of 60 seconds) was performed from above the metal mask using the plasma irradiation apparatus to prepare a film having a region of cell adhesiveness and cell proliferativity. Furthermore, 0.1 mL of an ethanol solution containing the block copolymer of Example 1 at a solid content concentration of 0.8 wt% was dropped onto this treated film and spin-coated (2000 rpm, 60 seconds) to form a coating layer (layer thickness: 40 nm) containing the block copolymer of Example 1, thereby obtaining a cell culture substrate having a two-layer structure of the layer containing the hydrophilic polymer and the coating layer containing the block copolymer of Example 1. This film was attached to the bottom surface of a bottomless 6-well plate to prepare a cell culture substrate with a bottom surface formed of a polycarbonate film.

### [Cell Culture and Evaluation of Cell Detachability on Cell Culture Substrate with Film-Formed Bottom Surface]

Cell culture and evaluation of cell detachability were carried out in the same manner as in Example 1 [Cell Culture and Evaluation of Cell Detachability], except that the cell culture substrate described above was used. The proportion of detached cell aggregates among the cell aggregates formed on the prepared cell culture substrate was 99.3%.

### [Change in Cell Detachment Method]

A cell culture substrate was prepared in the same manner as in Example 6 [Preparation of Cell Culture Substrate with Film-Formed Bottom Surface], except that the metal mask having a plurality of circular holes with a diameter of 0.2 mm was used. Cell detachability was evaluated by stirring at 300 rpm for 20 seconds using a shaker for microplates (manufactured by ISIS Co., Ltd.). The proportion of detached cell aggregates among the cell aggregates formed on the prepared cell culture substrate was 90.6%.

### [Evaluation of Relationship between Layer Thickness of Coating Layer Containing Block Copolymer and Cell Detachability]

After preparing a film having a region of cell adhesiveness and cell proliferativity by the method described in Example 6 [Preparation of Cell Culture Substrate with Film-Formed Bottom Surface], ethanol solutions of the block copolymer of Example 1 were prepared at 0.2 wt%, 0.4 wt%, 0.6 wt%, 0.8 wt%, and 1.0 wt%, respectively, and 0.1 mL of each was dropped and spin-coated (2000 rpm, 60 seconds) to obtain cell culture substrates having coating layers containing the block copolymer of 10 nm, 20 nm, 30 nm, 40 nm, or 50 nm, respectively. These cell culture substrates were attached to the bottom surface of a bottomless 6-well plate to prepare cell culture substrates having different layer thicknesses of the coating layer containing the block copolymer, with bottom surfaces formed of a polycarbonate film.

Cell culture and evaluation of cell detachability were carried out in the same manner as in Example 1 [Cell Culture and Evaluation of Cell Detachability], except that these cell culture substrates were used. The proportions of detached cell aggregates among the cell aggregates formed on the prepared cell culture substrates were 93.2%, 99.3%, 99.4%, 99.9%, and 99.9%, respectively.

### Example 7

### [Preparation of Cell Culture Substrate with Film-Formed Bottom Surface]

The cell culture substrate of Example 7 was prepared in the same manner as in Example 6 [Preparation of Cell Culture Substrate with Film-Formed Bottom Surface], except that the block copolymer of Example 2 was used.

### [Cell Culture and Evaluation of Cell Detachability on Cell Culture Substrate with Film-Formed Bottom Surface]

Cell culture and evaluation of cell detachability were carried out in the same manner as in Example 1 [Cell Culture and Evaluation of Cell Detachability], except that the cell culture substrate of Example 7 was used. The proportion of detached cell aggregates among the cell aggregates formed on the prepared cell culture substrate was 99.9%.

### Example 8

### [Preparation of Cell Culture Substrate with Film-Formed Bottom Surface]

The cell culture substrate of Example 8 was prepared in the same manner as in Example 6 [Preparation of Cell Culture Substrate with Film-Formed Bottom Surface], except that the block copolymer of Example 3 was used.

### [Cell Culture and Evaluation of Cell Detachability on Cell Culture Substrate with Film-Formed Bottom Surface]

Cell culture and evaluation of cell detachability were carried out in the same manner as in Example 1 [Cell Culture and Evaluation of Cell Detachability], except that the cell culture substrate of Example 8 was used. The proportion of detached cell aggregates among the cell aggregates formed on the prepared cell culture substrate was 99.0%.

### Example 9

### [Preparation of Cell Culture Substrate with Film-Formed Bottom Surface]

The cell culture substrate of Example 9 was prepared in the same manner as in Example 6 [Preparation of Cell Culture Substrate with Film-Formed Bottom Surface], except that the block copolymer of Example 4 was used.

### [Cell Culture and Evaluation of Cell Detachability on Cell Culture Substrate with Film-Formed Bottom Surface]

Cell culture and evaluation of cell detachability were carried out in the same manner as in Example 1 [Cell Culture and Evaluation of Cell Detachability], except that the cell culture substrate of Example 9 was used. The proportion of detached cell aggregates among the cell aggregates formed on the prepared cell culture substrate was 100.0%.

### Example 10

### [Preparation of Cell Culture Substrate with Film-Formed Bottom Surface]

The cell culture substrate of Example 10 was prepared in the same manner as in Example 6 [Preparation of Cell Culture Substrate with Film-Formed Bottom Surface], except that the block copolymer of Example 5 was used.

### [Cell Culture and Evaluation of Cell Detachability on Cell Culture Substrate with Film-Formed Bottom Surface]

Cell culture and evaluation of cell detachability were carried out in the same manner as in Example 1 [Cell Culture and Evaluation of Cell Detachability], except that the cell culture substrate of Example 10 was used. The proportion of detached cell aggregates among the cell aggregates formed on the prepared cell culture substrate was 100.0%.

### Example 11

### [Synthesis of Block Copolymer]

0.10 g (0.25 mmol) of 4-cyano-4-[(dodecylsulfanylthiocarbonyl)sulfanyl]pentanoic acid, 2.99 g (21 mmol) of n-butyl methacrylate, and 8 mg (0.05 mmol) of azobisisobutyronitrile were added to a test tube and dissolved in 18 mL of 1,4-dioxane. After degassing by nitrogen bubbling for 30 minutes, the mixture was reacted at 70°C for 17 hours to obtain an n-butyl methacrylate polymer solution. The conversion rate of n-butyl methacrylate at this stage was 78%.

21 mL (0.25 mmol) of the n-butyl methacrylate polymer solution, 11.32 g (100 mmol) of N-isopropylacrylamide, and 8 mg (0.05 mmol) of azobisisobutyronitrile were added to a 100 mL eggplant-shaped flask and dissolved in 10 mL of 1,4-dioxane. After degassing by nitrogen bubbling for 30 minutes, the mixture was reacted at 70°C for 3 hours. The conversion rate of N-isopropylacrylamide at this stage was 96%. After completion of the reaction, the reaction solvent was diluted with 70 mL of acetone, and poured into 3 L of hexane, and the precipitated solid was collected and dried under reduced pressure to prepare block copolymer B. Block copolymer B is also, like block copolymer A, a block copolymer containing a block segment (A) containing N-isopropylacrylamide as a monomer unit and a block segment (B) containing n-butyl methacrylate as a monomer unit.

The entire amount of the obtained block copolymer B and 0.82 g (5.0 mmol) of azobisisobutyronitrile were added to a 200 mL eggplant-shaped flask and dissolved in 112 mL of 1,4-dioxane. After degassing by nitrogen bubbling for 30 minutes, the mixture was reacted at 80°C for 3 hours. After completion of the reaction, the reaction solution was poured into 3 L of hexane, the precipitated solid was collected and dried under reduced pressure, then redissolved in 150 mL of acetone, poured into 5 L of pure water, and the precipitated gel-like product was collected and freeze-dried. This gave the block copolymer of Example 11.

The block copolymer of Example 11 is a copolymer in which a cyano group is introduced into the terminal on the block segment (A) side. The degree of polymerization of the block segment (A) in the block copolymer of Example 11 was 387. The Mn×10⁻³ of the block copolymer of Example 11 was 49.0, and the Mw/Mn was 1.20.

### [Measurement of Bubble Contact Angle]

A film coated with the block copolymer was prepared and the bubble contact angle was measured in the same manner as in Example 1 [Measurement of Bubble Contact Angle], except that the block copolymer of Example 11 was used. The bubble contact angle measured using the block copolymer of Example 11 was 160.8° at 21°C and 135.1° at 37°C, and the amount of change in the bubble contact angle was 25.6°.

### [Preparation of Cell Culture Substrate]

The cell culture substrate of Example 11 was prepared in the same manner as in Example 1 [Preparation of Cell Culture Substrate], except that the block copolymer of Example 11 was used.

### [Cell Culture and Evaluation of Cell Detachability]

Cell culture and evaluation of cell detachability were carried out in the same manner as in Example 1 [Cell Culture and Evaluation of Cell Detachability], except that the cell culture substrate of Example 11 was used. The proportion of detached cell aggregates among the cell aggregates formed on the prepared culture substrate was 98.2%.

### Example 12

### [Preparation of Cell Culture Substrate with Film-Formed Bottom Surface]

The cell culture substrate of Example 12 was prepared in the same manner as in Example 6 [Preparation of Cell Culture Substrate with Film-Formed Bottom Surface], except that the block copolymer of Example 11 was used.

### [Cell Culture and Evaluation of Cell Detachability on Cell Culture Substrate with Film-Formed Bottom Surface]

Cell culture and evaluation of cell detachability were carried out in the same manner as in Example 1 [Cell Culture and Evaluation of Cell Detachability], except that the cell culture substrate of Example 12 was used. The proportion of detached cell aggregates among the cell aggregates formed on the prepared cell culture substrate was 99.9%.

### [Evaluation of Relationship between Layer Thickness of Coating Layer Containing Block Copolymer and Cell Detachability]

Cell culture and evaluation of cell detachability were carried out in the same manner as in Example 6 [Evaluation of Relationship between Layer Thickness of Coating Layer Containing Block Copolymer and Cell Detachability], except that the block copolymer of Example 11 was used. The proportions of detached cell aggregates among the cell aggregates formed on the cell culture substrates having the block copolymer layer of Example 11 of 10 nm, 20 nm, 30 nm, 40 nm, or 50 nm, respectively, were 98.2%, 99.5%, 100.0%, 100.0%, and 99.9%, respectively.

### Comparative Example 4

### [Preparation of Cell Culture Substrate with Film-Formed Bottom Surface]

The cell culture substrate of Comparative Example 4 was prepared in the same manner as in Example 6 [Preparation of Cell Culture Substrate with Film-Formed Bottom Surface], except that the block copolymer of Comparative Example 1 was used.

### [Cell Culture and Evaluation of Cell Detachability on Cell Culture Substrate with Film-Formed Bottom Surface]

Cell culture and evaluation of cell detachability were carried out in the same manner as in Example 1 [Cell Culture and Evaluation of Cell Detachability], except that the cell culture substrate of Comparative Example 4 was used. The proportion of detached cell aggregates among the cell aggregates formed on the prepared cell culture substrate was 70.9%.

### Comparative Example 5

### [Measurement of Bubble Contact Angle]

0.1 mL of an 80 wt% ethanol aqueous solution containing a hydrophilic polymer at a solid content concentration of 0.6 wt% was dropped onto the polycarbonate film and spin-coated (2000 rpm, 60 seconds), and then the film was allowed to stand under a high-pressure mercury lamp for 1 hour to perform UV irradiation to cure the hydrophilic polymer, thereby forming a hydrophilic polymer layer (layer thickness: 75 nm). The polycarbonate film coated with the hydrophilic polymer film was immersed in water at 21°C or 37°C for 10 minutes, and the bubble contact angle (°) was measured using a contact angle meter DM300 manufactured by Kyowa Interface Science Co., Ltd. under conditions of in water and a bubble of 1 µL.

The bubble contact angle measured using the hydrophilic polymer was 145.0° at 21°C and 141.9° at 37°C, and the amount of change in the bubble contact angle was 3.1°.

### [Preparation of Cell Culture Substrate with Film-Formed Bottom Surface]

0.1 mL of an 80 wt% ethanol aqueous solution containing a hydrophilic polymer (manufactured by Toyo Gosei Co., Ltd., trade name: BIOSURFINE (registered trademark)-AWP) at a solid content concentration of 0.6 wt% was dropped onto the polycarbonate film and spin-coated (2000 rpm, 60 seconds), and then the film was allowed to stand under a high-pressure mercury lamp for 1 hour to perform UV irradiation to cure the hydrophilic polymer, thereby forming a hydrophilic polymer layer (layer thickness: 75 nm). The film coated with this hydrophilic polymer was covered with the metal mask having a plurality of circular holes with a diameter of 0.5 mm, and plasma treatment (under a gas pressure of 20 Pa, a conduction current of 20 mA, and an irradiation time of 60 seconds) was performed from above the metal mask using the plasma irradiation apparatus to prepare a film having a region of cell adhesiveness and cell proliferativity. This film was attached to the bottom surface of a bottomless 6-well plate to prepare the cell culture substrate of Comparative Example 5 with a bottom surface formed of a polycarbonate film.

### [Cell Culture and Evaluation of Cell Detachability on Cell Culture Substrate with Film-Formed Bottom Surface]

Cell culture and evaluation of cell detachability were carried out in the same manner as in Example 1 [Cell Culture and Evaluation of Cell Detachability], except that the cell culture substrate of Comparative Example 5 was used. The proportion of detached cell aggregates among the cell aggregates formed on the prepared cell culture substrate was 20.3%.

### Comparative Example 6

### [Synthesis of Block Copolymer]

0.10 g (0.25 mmol) of 4-cyano-4-[(dodecylsulfanylthiocarbonyl)sulfanyl]pentanoic acid, 5.69 g (40 mmol) of n-butyl methacrylate, and 8 mg (0.05 mmol) of azobisisobutyronitrile were added to a test tube and dissolved in 18 mL of 1,4-dioxane. After degassing by nitrogen bubbling for 30 minutes, the mixture was reacted at 70°C for 17 hours to obtain an n-butyl methacrylate polymer solution. The conversion rate of n-butyl methacrylate at this stage was 73%.

32 mL (0.25 mmol) of the n-butyl methacrylate polymer solution, 13.58 g (120 mmol) of N-isopropylacrylamide, and 8 mg (0.05 mmol) of azobisisobutyronitrile were added to a 100 mL eggplant-shaped flask and dissolved in 20 mL of 1,4-dioxane. After degassing by nitrogen bubbling for 30 minutes, the mixture was reacted at 70°C for 5 hours. The conversion rate of N-isopropylacrylamide at this stage was 85%. After completion of the reaction, the reaction solvent was diluted with 70 mL of acetone, and poured into 3 L of hexane. The precipitated solid was collected and dried under reduced pressure, then redissolved in 150 mL of acetone, and poured into 5 L of pure water, and the precipitated gel-like product was collected and freeze-dried. This gave the block copolymer of Comparative Example 6.

The block copolymer of Comparative Example 6 is a terminal-unmodified block copolymer in which a structure derived from the RAFT agent remains at the terminal. The degree of polymerization of the block segment (A) in the block copolymer of Comparative Example 6 was 408. The Mn×10⁻³ of the block copolymer of Comparative Example 6 was 52.3, and the Mw/Mn was 1.31.

### [Measurement of Bubble Contact Angle]

A film coated with the block copolymer was prepared and the bubble contact angle was measured in the same manner as in Example 1 [Measurement of Bubble Contact Angle], except that the block copolymer of Comparative Example 6 was used. The bubble contact angle measured using the block copolymer of Comparative Example 6 was 152.0° at 21°C and 150.3° at 37°C, and the amount of change in the bubble contact angle was 1.7°.

### [Preparation of Cell Culture Substrate]

The cell culture substrate of Comparative Example 6 was prepared in the same manner as in Example 1 [Preparation of Cell Culture Substrate], except that the block copolymer of Comparative Example 6 was used.

### [Cell Culture and Evaluation of Cell Detachability]

Cell culture and evaluation of cell detachability were carried out in the same manner as in Example 1 [Cell Culture and Evaluation of Cell Detachability], except that the cell culture substrate of Comparative Example 6 was used. The proportion of detached cell aggregates among the cell aggregates formed on the prepared culture substrate was 57.4%.

### Comparative Example 7

### [Preparation of Cell Culture Substrate with Film-Formed Bottom Surface]

The cell culture substrate of Comparative Example 7 was prepared in the same manner as in Example 6 [Preparation of Cell Culture Substrate with Film-Formed Bottom Surface], except that the block copolymer of Comparative Example 6 was used.

### [Cell Culture and Evaluation of Cell Detachability on Cell Culture Substrate with Film-Formed Bottom Surface]

Cell culture and evaluation of cell detachability were carried out in the same manner as in Example 1 [Cell Culture and Evaluation of Cell Detachability], except that the cell culture substrate of Comparative Example 7 was used. The proportion of detached cell aggregates among the cell aggregates formed on the prepared cell culture substrate was 58.9%.

### <Results>

Table 1 shows the measurement results of the amount of change in the bubble contact angle and the detachment rate measured using the block copolymers or cell culture substrates of Examples 1 to 5 and 11 and Comparative Examples 1 to 3 and 6. Table 2 shows the measurement results of the detachment rate measured using the cell culture substrates of Examples 6 to 10 and 12 and Comparative Examples 4 to 5 and 7. In the cell culture substrates of the Examples, the coating layer had a phase-separated structure of the block copolymer at the culture temperature (37°C).

The detachment rate 1 in Table 1 refers to the detachment rate calculated by forming cell aggregates in a polystyrene dish and counting the number of detached cell aggregates. The detachment rate 2 in Tables 2 to 4 refers to the detachment rate calculated by forming cell aggregates in a plate having a polycarbonate film on its bottom surface and counting the number of detached cell aggregates.

**[Table 1]**

| | Amount of change in bubble contact angle | Block segment (A) | Detachment rate 1 |
|---|---|---|---|
| | | Degree of polymeri zation | |
| Example 1 | 11.1° | 238 | 79.4% |
| Example 2 | 32.3° | 238 | 85.0% |
| Example 3 | 23.7° | 238 | 88.4% |
| Example 4 | 26.6° | 238 | 89.6% |
| Example 5 | 24.1° | 238 | 80.3% |
| Example 11 | 25.6° | 387 | 98.2% |
| Comparative Example 1 | 4.3° | 238 | 44.2% |
| Comparative Example 2 | | | 3.1% |
| Comparative Example 3 | 1.8° | | |
| Comparative Example 6 | 1.7° | 408 | 57.4% |

**[Table 2]**

| | Amount of change in bubble contact angle | Block segment (A) | Detachment rate 2 |
|---|---|---|---|
| | | Degree of polymerization | |
| Example 6 | 11.1° | 238 | 99.3% |
| Example 7 | 32.3° | 238 | 99.9% |
| Example 8 | 23.7° | 238 | 99.0% |
| Example 9 | 26.6° | 238 | 100.0% |
| Example 10 | 24.1° | 238 | 100.0% |
| Example 12 | 25.6° | 387 | 99.9% |
| Comparative Example 4 | 4.3° | 238 | 70.9% |
| Comparative Example 5 | 3.1° | | 20.3% |
| Comparative Example 7 | 1.7° | 408 | 58.9% |

**[Table 3]**

| Polymer used: Example 1 | | | |
|---|---|---|---|
| Polymer concentration (wt%) | Layer thickness (nm) | Amount of change in bubble contact angle (°) | Detachment rate 2 (%) |
| 0.2 | 10 | | 93.2% |
| 0.4 | 20 | | 99.3% |
| 0.6 | 30 | 11.1 | 99.4% |
| 0.8 | 40 | | 99.9% |
| 1.0 | 50 | | 99.9% |

**[Table 4]**

| Polymer used: Example 11 | | | |
|---|---|---|---|
| Polymer concentration (wt%) | Layer thickness (nm) | Amount of change in bubble contact angle (°) | Detachment rate 2 (%) |
| 0.2 | 10 | | 98.2% |
| 0.4 | 20 | | 99.5% |
| 0.6 | 30 | 25.6 | 100.0% |
| 0.8 | 40 | | 100.0% |
| 1.0 | 50 | | 99.9% |

As shown in Tables 1 and 2, it was demonstrated that the cell detachment rate is improved when the amount of change in the bubble contact angle is 5.0° or more.

As shown in Tables 3 and 4, it was confirmed that the cell detachment rate is improved when the amount of change in the bubble contact angle is 5.0° or more even when the film thickness is changed.

## Claims

1. A temperature-responsive block copolymer comprising:
a block segment (A) having a lower critical solution temperature (LCST) in water; and
a water-insoluble block segment (B),
wherein, when a film comprising the temperature-responsive block copolymer is formed, an amount of change in a bubble contact angle on a surface of the film, as measured under respective conditions in water at 21°C and in water at 37°C, is 5.0° or more.

2. The temperature-responsive block copolymer according to claim 1, wherein the amount of change in the bubble contact angle is 10° or more and 40° or less.

3. The temperature-responsive block copolymer according to claim 1 or 2, wherein at least one functional group selected from the group consisting of a hydroxyl group, a mercapto group, an amino group, a substituted amino group, a cyano group, an ester group, an amide group, a carbonyl group, and a carboxyl group is introduced into at least one terminal of the block segment (A) or the block segment (B).

4. The temperature-responsive block copolymer according to claim 1 or 2, wherein at least one functional group selected from the group consisting of a hydroxyl group, a mercapto group, an amino group, a substituted amino group, a cyano group, an ester group, an amide group, a carbonyl group, and a carboxyl group is introduced into a terminal on the block segment (A) side.

5. The block copolymer according to claim 1 or 2, wherein the block segment (B) comprises a monomer unit represented by the following General Formula (b1). 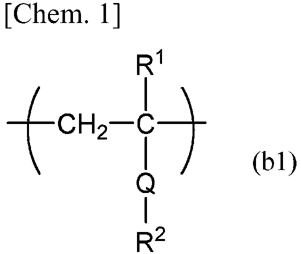 [In the formula, R¹ represents a hydrogen atom or a methyl group, Q represents an ester group, and R² represents a hydrocarbon group having 1 to 30 carbon atoms.]

6. The temperature-responsive block copolymer according to claim 1 or 2, wherein a degree of polymerization of the block segment (A) is 100 or more and less than 400.

7. A cell culture substrate comprising a substrate and a coating layer covering at least a part of a surface of the substrate,
wherein the coating layer comprises a temperature-responsive block copolymer,
the temperature-responsive block copolymer comprises a block segment (A) having a lower critical solution temperature (LCST) in water and a water-insoluble block segment (B), and
an amount of change in a bubble contact angle on a surface of the coating layer, as measured under respective conditions in water at 21°C and in water at 37°C, is 5.0° or more.

8. The cell culture substrate according to claim 7, wherein a layer thickness of the coating layer is 5 to 1000 nm.

9. The cell culture substrate according to claim 7 or 8, wherein the substrate is any one of a plate, a film, a flask, and a bag.

10. The cell culture substrate according to claim 7 or 8, comprising a region having cell adhesiveness and temperature-responsive detachability, and a region of non-cell adhesiveness.

11. The cell culture substrate according to claim 10, wherein the region of non-cell adhesiveness comprises a hydrophilic polymer.

12. The cell culture substrate according to claim 7 or 8, wherein a thickness of the coating layer is 10 to 50 nm.

13. The cell culture substrate according to claim 7 or 8, wherein the substrate is polycarbonate.

14. A method for producing a temperature-responsive block copolymer comprising a block segment (A) having a lower critical solution temperature (LCST) in water and a water-insoluble block segment (B), wherein a polar group is introduced into a terminal of the block segment (A) or (B), the method comprising:
a step of reacting a thiocarbonylthio group-containing block copolymer comprising the block segments (A) and (B), and a terminal bonded to the block segment (A) or (B) comprising a thiocarbonylthio group represented by the following Formula (I): 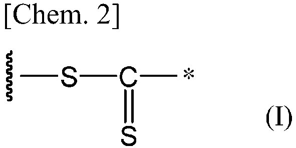
[In Formula (I), a wavy line indicates a bond on the block segment (A) or (B) side, and * indicates a bond on a terminal side.].
with a radical generator having a polar group to convert the terminal comprising the thiocarbonylthio group into a terminal comprising the polar group, thereby obtaining the temperature-responsive block copolymer.

15. The method according to claim 14, wherein the radical generator having a polar group comprises at least one selected from the group consisting of azobisisobutyronitrile, 2,2'-azobis[2-methyl-N-(2-hydroxyethyl)propionamide], 4,4'-azobis-(4-cyanovaleric acid), and 2,2'-azobis-(2-methylpropionic acid).
